# EUROPÄISCHE PATENTSCHRIFT

(11) **EP 3 827 745 B1**
(45) Veröffentlichungstag und Bekanntmachung des Hinweises auf die Patenterteilung: **24.06.2026**
(21) Anmeldenummer: 20202947.6
(22) Anmeldetag: 21.10.2020
(51) Int. Cl.: A61B 5/055, A61B 5/00, G01R 33/54, A61B 5/107

(54) **VORRICHTUNG ZUR ERSTELLUNG EINER GEBISSÜBERSICHTSKARTE**
DEVICE FOR PRODUCING A DENTAL CHART
DISPOSITIF DE CRÉATION D'UNE CARTE SYNOPTIQUE DE LA DENTITION

(30) Priorität: 27.11.2019 US 201962941179 P
(43) Veröffentlichungstag der Anmeldung: 02.06.2021
(73) Patentinhaber: Siemens Healthineers AG, 91301 Forchheim (DE)
(72) Erfinder: Lauer, Lars, 91077 Neunkirchen (DE); Greiser, Andreas, 91054 Erlangen (DE); Kartmann, René, 90491 Nürnberg (DE); Grodzki, David, 91058 Erlangen (DE); Zeller, Mario, 91054 Erlangen (DE)
(74) Vertreter: Siemens Healthineers Patent Attorneys

(56) Entgegenhaltungen:
- US-A1- 2007 030 346
- US-A1- 2013 252 196
- US-A1- 2014 084 920
- US-A1- 2015 305 669
- US-A1- 2019 147 648
- TYMOFIYEVA OLGA ET AL: "High-resolution 3D magnetic resonance imaging and quantification of carious lesions and dental pulp in vivo", vol. 22, no. 6, 19 November 2009 (2009-11-19), DE, GB, pages 365 - 374, XP055792816, ISSN: 0968-5243, Retrieved from the Internet <URL:http://link.springer.com/article/10.1007/s10334-009-0188-9/fulltext.html> [retrieved on 20210406], DOI: 10.1007/s10334-009-0188-9
- BRACHER ANNA-KATINKA ET AL: "Feasibility of ultra-short echo time (UTE) magnetic resonance imaging for identification of carious lesions : MRI for Identification of Carious Lesions", vol. 66, no. 2, 28 February 2011 (2011-02-28), US, pages 538 - 545, XP055792794, ISSN: 0740-3194, Retrieved from the Internet <URL:https://api.wiley.com/onlinelibrary/tdm/v1/articles/10.1002%2Fmrm.22828> [retrieved on 20210406], DOI: 10.1002/mrm.22828
- CANKAR KSENIJA ET AL: "T2 Mapping as a Tool for Assessment of Dental Pulp Response to Caries Progression: An in vivo MRI Study", vol. �54, no. 1, 11 September 2019 (2019-09-11), CH, pages 24 - 35, XP055792578, ISSN: 0008-6568, Retrieved from the Internet <URL:https://www.karger.com/Article/Pdf/501901> [retrieved on 20210406], DOI: 10.1159/000501901

## Beschreibung

Die Erfindung betrifft ein Verfahren zur Erstellung einer Gebissübersichtskarte von einem Gebiss eines Untersuchungsobjekts. Ferner betrifft die Erfindung eine Magnetresonanzvorrichtung mit einer Recheneinheit sowie ein Computerprogrammprodukt, welches direkt in einen Datenspeicher einer Recheneinheit einer Magnetresonanzvorrichtung ladbar ist, um ein erfindungsgemäßes Verfahren auszuführen.

Erkrankungen der Zähne und des Parodontiums, wie z. B. Karies oder Parodontitis, werden heutzutage üblicherweise mit röntgenbasierten Bildgebungsverfahren diagnostiziert. Dabei kommen vor allem konventionelle oder digitale Röntgen-Projektionsverfahren, sowie kürzlich auch dreidimensionale Röntgenverfahren, zum Einsatz. Ein Beispiel für ein dreidimensionales Röntgenverfahren stellt die digitale Volumentomographie dar, welche für eine Bildgebung von Zähnen und des Viscerocraniums eingesetzt werden kann.

Ein großer Nachteil von Röntgenverfahren ist die Notwendigkeit des Einsatzes von ionisierender Strahlung für die Bildgebung. Ein Bildgebungsverfahren, welches ionisierende Strahlen vermeidet, stellt die Magnetresonanztomografie dar. Diese ermöglicht typischerweise einen besseren Weichgewebekontrast als Röntgenverfahren und unterstützt standardmäßig eine dreidimensionale Bildgebung eines Untersuchungsobjekts. Die Magnetresonanztomografie stellt somit eine potentielle Alternative zu bekannten Röntgenverfahren bei der Bildgebung eines Gebisses und/oder eines Kieferbereichs sowie der Diagnose von Zahnerkrankungen des Untersuchungsobjekts dar.

Die Magnetresonanztomografie ist ein bekanntes Bildgebungsverfahren (vg1. TYMOFIYEVA OLGA ET AL: "High-resolution 3D magnetic resonance imaging and quantification of carious lesions and dental pulp in vivo", DOI: 10.1007/s10334-009-0188-9, oder US 2019/147648 A1), mit welchem Magnetresonanzbilder eines Inneren des Untersuchungsobjekts erzeugt werden können. Zur Durchführung einer Magnetresonanzmessung wird das Untersuchungsobjekt üblicherweise in einem starken, statischen und homogenen Grundmagnetfeld (B0-Feld) einer Magnetresonanzvorrichtung positioniert. Das Grundmagnetfeld kann magnetische Feldstärken von 0,2 Tesla bis 7 Tesla aufweisen, so dass sich Kernspins des Untersuchungsobjekts entlang des Grundmagnetfeldes ausrichten. Um sogenannte Kernspinresonanzen auszulösen, werden hochfrequente Anregungsimpulse in das Untersuchungsobjekt eingestrahlt. Jeder hochfrequente Anregungsimpuls bewirkt eine Abweichung einer Magnetisierung bestimmter Kernspins des Untersuchungsobjekts von dem Grundmagnetfeld um einen Betrag, welcher auch als Flipwinkel bekannt ist. Ein hochfrequenter Anregungsimpuls kann dabei ein magnetisches Wechselfeld mit einer Frequenz aufweisen, welche der Larmorfrequenz bei der jeweiligen statischen Magnetfeldstärke entspricht. Die angeregten Kernspins können eine rotierende und abklingende Magnetisierung (Kernspinresonanz) aufweisen, welche sich mittels spezieller Antennen erfassen lässt. Zur räumlichen Kodierung der Kernspinresonanzen des Untersuchungsobjekts können dem Grundmagnetfeld magnetische Gradientenfelder überlagert werden.

Die empfangenen Kernspinresonanzen werden typischerweise digitalisiert und als komplexe Werte in einer k-Raum-Matrix gespeichert. Diese k-Raum-Matrix kann als Grundlage für die Rekonstruktion von Magnetresonanzbildern sowie einer Bestimmung von Spektroskopiedaten verwendet werden. Die Rekonstruktion eines Magnetresonanzbilds erfolgt typischerweise mittels einer mehrdimensionalen Fourier-Transformation der k-Raum-Matrix.

Die Magnetresonanztomografie eignet sich aufgrund der Vermeidung von ionisierender Strahlung insbesondere für eine kontinuierliche diagnostische Überwachung von Zahnerkrankungen und/oder einer Zahnentwicklung im Rahmen einer longitudinalen Bildgebungsstudie. Bei longitudinalen Bildgebungsstudien wird üblicherweise eine Mehrzahl von Bildgebungsuntersuchungen durchgeführt, um eine Progression einer Erkrankung oder einen Erfolg einer therapeutischen Behandlung über einen vorbestimmten Zeitraum zu bestimmen. Ein Nachteil der Magnetresonanztomografie stellt dabei jedoch eine erhöhte Zeitdauer dar, welche üblicherweise mit der Aufnahme von Magnetresonanzdaten des Untersuchungsobjekts verbunden ist. Dies kann insbesondere bei pädiatrischen Patienten ein Problem darstellen, da Bewegungen des Patienten während der Bildgebungsuntersuchung Bildartefakte verursachen können, welche die Qualität der Magnetresonanzbilder beeinträchtigen. Ferner kann die erhöhte Zeitdauer, welche mit der Erfassung von Magnetresonanzdaten verbunden ist, ein Problem für die Behandlung einer Vielzahl von Patienten darstellen, welche üblicherweise von zahnmedizinischen Einrichtungen behandelt werden.

Es ist daher eine Aufgabe der Erfindung, eine Effizienz der Bildgebung eines Gebisses eines Untersuchungsobjekts zu erhöhen.

Diese Aufgabe wird durch die Gegenstände der unabhängigen Patentansprüche erfindungsgemäß gelöst. Vorteilhafte Ausführungsformen und zweckmäßige Weiterbildungen sind Gegenstand der Unteransprüche.

Bei dem erfindungsgemäßen Verfahren wird eine Gebissübersichtskarte von einem Gebiss eines Untersuchungsobjekts anhand von Magnetresonanzdaten einer Magnetresonanzmessung des Gebisses erstellt. Das Gebiss des Untersuchungsobjekts kann in Abhängigkeit einer Voraussetzung des Untersuchungsobjekts einen Teil eines Zahns, einen Zahnzwischenraum, einen Zahn, mehrere Zähne, einen Zahnbogen oder mehrere Zahnbögen umfassen. Ein Zahnbogen weist typischerweise einen Zahnhalteapparat oder einen Teil des Zahnhalteapparats mit allen Zähnen oder einem Teil der Zähne eines Oberkiefers oder eines Unterkiefers des Untersuchungsobjekts auf. Vorzugsweise umfasst das Gebiss alle Zähne des Oberkiefers und des Unterkiefers des Untersuchungsobjekts.

Eine Magnetresonanzmessung stellt vorzugsweise eine Aufnahme von Kernspinresonanzen des Untersuchungsobjekts mittels einer Magnetresonanzvorrichtung dar. Die aufgenommenen Kernspinresonanzen können digitalisiert und als k-Raum-Matrix, den sogenannten Magnetresonanzdaten, abgespeichert werden. Es ist vorstellbar, dass die Magnetresonanzmessung in Abhängigkeit von Bildgebungsparametern veränderbar ist. Beispielsweise lassen sich eine Position eines Bildgebungsvolumens, eine Abmessung des Bildgebungsvolumens, eine Anregedauer, eine Echozeit, eine Repititionszeit oder weitere Bildgebungsparameter der Magnetresonanzmessung anpassen, um Magnetresonanzdaten eines diagnostisch relevanten Bereichs des Gebisses zu erfassen. Die Bildgebungsparameter der Magnetresonanzmessung können in einer sogenannten Bildgebungssequenz zusammengefasst sein, welche einen Ablauf der Magnetresonanzmessung, wie z. B. eine Anzahl und eine zeitliche Abfolge von hochfrequenten Anregungsimpulsen, bestimmen kann. Es ist vorstellbar, dass die Magnetresonanzdaten zur Rekonstruktion von Magnetresonanzbildern des Gebisses des Untersuchungsobjekt verwendet werden.

In einem Schritt des erfindungsgemäßen Verfahrens wird eine Magnetresonanzmessung zur Erfassung von Magnetresonanzdaten des Gebisses durchgeführt, wobei ein Bildgebungsvolumen der Magnetresonanzmessung mit einem Volumen des Gebisses abgestimmt ist und wobei das Bildgebungsvolumen eine Anzahl von Zähnen des Gebisses umfasst. Ein Bildgebungsvolumen kann ein beliebig geformtes Volumen innerhalb einer Bildaufnahmeregion der Magnetresonanzvorrichtung darstellen, aus welchem Kernspinresonanzen des Untersuchungsobjekts empfangen werden können. Vorzugsweise wird das Untersuchungsobjekt für die Magnetresonanzmessung so in der Bildaufnahmeregion der Magnetresonanzvorrichtung positioniert, dass zumindest ein Teil eines Volumens des Gebisses des Untersuchungsobjekts mit dem Bildgebungsvolumen übereinstimmt. Das Bildgebungsvolumen ist dabei insbesondere mit dem Volumen des Gebisses abgestimmt, sodass ein diagnostisch relevanter Bereich des Gebisses innerhalb des Bildgebungsvolumens positioniert ist. In einem Beispiel kann das Bildgebungsvolumen so mit dem Volumen des Gebisses abgestimmt sein, dass eine Anzahl von Zähnen, beispielsweise ein Zahn, zwei Zähne, drei Zähne oder eine Mehrzahl von Zähnen, von dem Bildgebungsvolumen umfasst sind. Die Anzahl von Zähnen kann dabei einen Zahn oder eine Mehrzahl von Zähnen des Oberkiefers und/oder des Unterkiefers umfassen. Das Bildgebungsvolumen kann eine beliebige Form aufweisen. In einer bevorzugten Ausführungsform weist das Bildgebungsvolumen eine sphärische oder eine ovoide Form auf. Es ist aber ebenso vorstellbar, dass das Bildgebungsvolumen eine polygonale Form besitzt oder sich aus einer Kombination einer ovoiden Form und einer polygonalen Form ergibt. Wie oben beschrieben, wird bei dem Erfassen der Magnetresonanzdaten des Gebisses eine k-Raum-Matrix mit den Kernspinresonanzen der Anzahl von Zähnen aufgenommen, welche in dem Bildgebungsvolumen positioniert sind.

In einem weiteren Schritt des erfindungsgemäßen Verfahrens wird zur Bestimmung einer Auffälligkeit eine Analyse von Abschnitten des Gebisses, welche jeweils eine Teilmenge der Anzahl von Zähnen des Gebisses umfassen, anhand der Magnetresonanzdaten durchgeführt, wobei in zumindest einem Abschnitt eine Auffälligkeit bestimmt wird. Ein Abschnitt kann eine beliebige Teilmenge der Anzahl von Zähnen des Gebisses darstellen. Vorzugsweise umfasst ein Abschnitt genau einen Zahn, genau zwei Zähne oder eine vorbestimmte Anzahl von Zähnen des Gebisses. Es ist jedoch ebenso vorstellbar, dass ein Abschnitt einen Teil eines Zahns darstellt. Beispielsweise kann ein Abschnitt durch ein Raster definiert sein, welches das Volumen des Gebisses in gleichmäßige oder ungleichmäßige Rasterelemente unterteilt. Ein Abschnitt kann dabei genau ein Rasterelement oder eine Mehrzahl von Rasterelementen darstellen. In einem anderen Beispiel kann ein Abschnitt ein kleinstes auflösbares Bildelement (Pixel) oder ein Vielfaches eines Bildelements eines Magnetresonanzbilds darstellen, welches aus den Magnetresonanzdaten rekonstruiert wird. Vorzugsweise wird das Volumen des Gebisses in eine Mehrzahl von Abschnitten untereilt, um die Bestimmung von Auffälligkeiten und/oder eine weitere Verarbeitung und/oder Darstellung der Auffälligkeiten zu vereinfachen. Die Durchführung der Analyse des Gebisses erfolgt dabei insbesondere abschnittsweise, bis alle Abschnitte des Gebisses analysiert sind.

Es ist ebenso vorstellbar, dass die Analyse der Abschnitte des Gebisses anhand von Magnetresonanzbildern erfolgt, welche in Abhängigkeit der Magnetresonanzdaten des Gebisses rekonstruiert werden. Die Bestimmung von Auffälligkeiten kann beispielsweise in Abhängigkeit von Kontrasten einzelner oder mehrerer Bildelemente eines Magnetresonanzbilds erfolgen. Es ist ebenso vorstellbar, dass aus den Kontrasten mehrerer Bildelemente charakteristische Strukturen abgeleitet werden, welche sich einer anatomischen Struktur und/oder einer Auffälligkeit zuordnen lassen. Vorzugsweise wird für die Bestimmung von Auffälligkeiten eine Bildverarbeitungseinheit eingesetzt. Die Bildverarbeitungseinheit kann dazu ausgebildet sein, charakteristische Strukturen anhand von Kontrasten von Bildelementen eines Magnetresonanzbilds und/oder von Volumenelementen (Voxel) eines dreidimensionalen Datensatzes von Magnetresonanzbildern automatisch zu identifizieren. Die Bestimmung von Auffälligkeiten kann ferner einen Einsatz eines intelligenten Algorithmus, wie **z. B.** eines neuronalen Netzes, eines Expertensystems, eines Optimierungsverfahrens, einer Methode des Deep Learning oder dergleichen umfassen. Intelligente Algorithmen können dazu ausgelegt sein, Auffälligkeiten in Abhängigkeit von weiteren Informationen, wie **z. B.** Trainingsbildern, Magnetresonanzbildern mit klassifizierten Auffälligkeiten sowie Kontrastvorlagen mit typischen Kontrasten oder Referenzwerten von Auffälligkeiten, zu bestimmen. Mittels der Analyse der Abschnitte des Gebisses lassen sich quantitative und/oder qualitative Informationen über einen Zahn und/oder die Anzahl von Zähnen bestimmen. Beispielsweise können die Informationen eine Aussage darüber enthalten, welche Zähne von einer Zahnerkrankung betroffen sind und/oder welches Ausmaß die Zahnerkrankung aufweist. In zumindest einem Abschnitt des Gebisses wird dabei eine Auffälligkeit bestimmt. Aus den Informationen aller Abschnitte des Gebisses kann weiterhin ein Zustand des Gebisses des Untersuchungsobjekts abgeleitet werden.

In einem weiteren Schritt des erfindungsgemäßen Verfahrens wird eine Gebissübersichtskarte in Abhängigkeit der Magnetresonanzdaten und der Auffälligkeit des zumindest einen Abschnitts des Gebisses erstellt, wobei die Gebissübersichtskarte eine Repräsentation eines Zahns des Gebisses des Untersuchungsobjekts und eine Repräsentation der Auffälligkeit des zumindest einen Abschnitts des Gebisses aufweist. Vorzugsweise umfasst die Gebissübersichtskarte eine geordnete Darstellung der Anzahl von Zähnen des Gebisses des Untersuchungsobjekts. Ein Zahn kann dabei zum Beispiel in Abhängigkeit einer relativen Position zu einem Zahnbogen und/oder zu weiteren Zähnen des Untersuchungsobjekts aufgetragen werden und eine Nummerierung und/oder Kennzeichnung aufweisen, welche eine Information über einen Typ und/oder die Position des Zahns in dem Gebiss des Untersuchungsobjekts angeben.

Die Gebissübersichtskarte weist insbesondere eine Repräsentation eines Zahns oder der Anzahl von Zähnen auf. Eine Repräsentation kann eine beliebige, schematisierte Darstellung eines Zahns und/oder der Anzahl von Zähnen umfassen. Es ist weiterhin vorstellbar, dass die Gebissübersichtskarte Magnetresonanzbilder oder Ausschnitte von Magnetresonanzbildern eines Zahns und/oder der Anzahl von Zähnen umfasst. Vorzugsweise wird die Information über den Typ und/oder die Position des Zahns in dem Gebiss anhand von Kontrasten oder Signalintensitäten der Bildelemente der Magnetresonanzbilder bestimmt. Die Magnetresonanzbilder können weiterhin mit der Repräsentation der Anzahl von Zähnen und/oder der Nummerierung und/oder der Kennzeichnung verknüpft werden.

Die Gebissübersichtskarte weist ferner eine Repräsentation der Auffälligkeit des zumindest einen Abschnitts des Gebisses auf. Eine solche Repräsentation kann beispielsweise eine Markierung und/oder eine farbliche Hervorhebung der Auffälligkeit in den Magnetresonanzbildern der Anzahl von Zähnen umfassen. Es ist aber ebenso vorstellbar, dass die Repräsentation der Auffälligkeit einen Hinweis, wie **z. B.** ein Textfeld, ein geometrisches Objekt, eine numerische Ziffer, ein Symbol, eine Markierung oder eine beliebige Kombination solcher Hinweise, umfasst. In einem Beispiel werden ein Zahn mit einer Auffälligkeit und/oder eine Auffälligkeit des Zahns auf der Gebissübersichtskarte mit einem Hinweis versehen, welcher eine Information über den Typ des Zahns, eine vorliegende Zahnerkrankung und/oder ein Ausmaß der Zahnerkrankung, wie **z. B.** ein Fortschrittsstadium der Zahnerkrankung, anzeigt und/oder kodiert. In einer bevorzugten Ausführungsform werden Abschnitte des Gebisses mit einer Auffälligkeit bei dem Erstellen der Gebissübersichtskarte markiert und/oder hervorgehoben. Eine Auffälligkeit kann dabei mit einem Hinweis versehen werden, welcher eine Information über die Auffälligkeit enthält.

In einem Schritt des erfindungsgemäßen Verfahrens wird die Gebissübersichtskarte bereitgestellt. Das Bereitstellen kann **z. B.** eine Ausgabe der Gebissübersichtskarte auf einer beliebigen Anzeigeeinheit umfassen. Es ist vorstellbar, dass die Gebissübersichtskarte auf einem Bildschirm der Magnetresonanzvorrichtung ausgegeben wird. Eine Ausgabe der Übersichtskarte auf einem Bildschirm der Magnetresonanzvorrichtung kann einen behandelnden Mediziner, wie **z. B.** einen Zahnarzt oder einen Kieferchirurgen, dabei unterstützen, den Zustand des Gebisses des Untersuchungsobjekts zu beurteilen. Beispielsweise kann die Gebissübersichtskarte eine schematisierte Repräsentation von Zähnen mit Auffälligkeiten enthalten, welche dem behandelnden Mediziner einen Anhaltspunkt geben, bei welchen Zähnen eine genauere Untersuchung erforderlich ist. Ein Beispiel hierfür ist die schematisierte Darstellung einer Zahnkaries im Anfangsstadium eines Zahns. Die Gebissübersichtskarte kann den behandelnden Mediziner ebenso auf Auffälligkeiten hinweisen, welche durch eine rein visuelle Inspektion des Gebisses schwierig zu diagnostizieren sind. Solche Auffälligkeiten können beispielsweise Zysten oder Entzündungen im Bereich einer Zahnwurzel eines Zahns darstellen. Die Gebissübersichtskarte kann ferner auf einem Bildschirm eines Mobilgeräts, wie **z. B.** einem Smartphone oder einem Tablet, ausgegeben werden. In einer Ausführungsform ist das Untersuchungsobjekt ein Patient, wobei die Gebissübersichtskarte im Rahmen einer Information des Patienten und/oder einer Dokumentation des Zustands des Gebisses des Patienten auf ein privates Mobilgerät des Patienten übertragen wird. Das Mobilgerät kann eine entsprechende Applikation oder Software-Anwendung aufweisen, welche dazu ausgebildet ist, die Gebissübersichtskarte mittels eines Bildschirms des Mobilgeräts an einen Nutzer des Mobilgeräts auszugeben. Die Applikation kann ferner eine Anleitung zur Unterstützung einer Zahnreinigung aufweisen und/oder mit einer entsprechenden Applikation zur Unterstützung der Zahnreinigung gekoppelt sein. Dadurch kann der Nutzer des Mobilgeräts auf vorteilhafte Weise über den Zustand des Gebisses informiert und/oder auf einen besonderen Bedacht bei der Reinigung von Zahnregionen mit einer Auffälligkeit hingewiesen werden.

Es ist ebenso vorstellbar, dass das Bereitstellen der Gebissübersichtskarte eine Speicherung der Gebissübersichtskarte auf einem Datenspeicher umfasst. Der Datenspeicher kann dazu ausgebildet sein, eine Mehrzahl von Gebissübersichtskarten zu speichern und dem behandelnden Mediziner und/oder dem Untersuchungsobjekt zur Verfügung zu stellen. In einer Ausführungsform weist die Magnetresonanzvorrichtung eine Recheneinheit auf, welche dazu ausgebildet ist, eine erste Gebissübersichtskarte oder eine Mehrzahl von Gebissübersichtskarten des Untersuchungsobjekts aus einem Datenspeicher abzurufen und eine Magnetresonanzmessung in Abhängigkeiten der Gebissübersichtskarte oder der Mehrzahl von Gebissübersichtskarten zu parametrieren. Die Parametrierung der Magnetresonanzmessung kann beispielsweise eine Einstellung einer Bildgebungssequenz oder eines Bildgebungsparameters einer nachfolgenden Magnetresonanzmessung umfassen.

Das erfindungsgemäße Verfahren kann den Vorteil bieten, dass eine effiziente und reproduzierbare Bildgebung des Gebisses eines Untersuchungsobjekts mittels einer Magnetresonanzvorrichtung ermöglicht wird. Weiterhin ist der Vorteil denkbar, dass durch eine automatische Ermittlung von Auffälligkeiten der Anzahl von Zähnen sowie die Erstellung einer Gebissübersichtskarte zur Dokumentation des Zustands des Gebisses eine Zeitdauer und/oder ein Aufwand für die Erstellung von Daten, welche für eine quantitative und qualitative Beurteilung des Zustands des Gebisses erforderlich sind, auf vorteilhafte Weise sich reduzieren lassen.

In einer möglichen Ausführungsform des erfindungsgemäßen Verfahrens ist der zumindest eine Abschnitt, in welchem die Auffälligkeit bestimmt wird, ein erster Abschnitt, wobei bei dem Durchführen der Analyse von Abschnitten des Gebisses ein Vorliegen einer Auffälligkeit in einem zweiten Abschnitt ausgeschlossen wird. Ein Ausschluss des Vorliegens einer Auffälligkeit kann bedeuten, dass die Teilmenge der Anzahl von Zähnen des zweiten Abschnitts keine Auffälligkeiten aufweist. Die Teilmenge der Anzahl von Zähnen des zweiten Abschnitts kann somit einen gesunden Zahn aufweisen. Es ist insbesondere vorstellbar, dass die Teilmenge der Anzahl von Zähnen des zweiten Abschnitts keine für eine Zahnerkrankung, eine Zahnfehlstellung und/oder für eine Zahnbeschädigung typische Auffälligkeit aufweisen. Vorzugsweise wird der erste Abschnitt mit der Auffälligkeit bei der Erstellung der Gebissübersichtskarte gegenüber dem zweiten Abschnitt hervorgehoben. Dies kann **z. B.** mittels eines Hinweises, einer Kennzeichnung und/oder einer farblichen Markierung erfolgen.

Durch den Ausschluss von Auffälligkeiten in einem oder mehreren Abschnitten des Gebisses kann die Beurteilung des Zustands des Gebisses des Untersuchungsobjekts auf vorteilhafte Weise beschleunigt werden. Ferner lässt sich der Zustand des Gebisses anhand von Informationen über Abschnitte mit Auffälligkeiten und Abschnitte ohne Auffälligkeiten mittels der Gebissübersichtskarte dokumentieren. Auf dieser Basis kann auf vorteilhafte Weise eine Planungsgrundlage für nachfolgende Bildgebungsuntersuchungen, z. B. im Rahmen einer langfristig angelegten Therapie von Zahnkaries, einer kieferorthopädischen Maßnahme und/oder einer longitudinalen Bildgebungsstudie, bereitgestellt werden.

In einer möglichen Ausführungsform des erfindungsgemäßen Verfahrens umfasst das Durchführen der Analyse von Abschnitten des Gebisses eine Bestimmung einer Entzündung und/oder einer Zahnkaries. Für die Bestimmung einer Entzündung können ein Bildgebungsparameter und/oder eine Bildgebungssequenz der Magnetresonanzmessung so eingestellt werden, dass ein hoher Weichgewebekontrast der Magnetresonanzdaten und/oder der Magnetresonanzbilder erhalten wird. Dadurch lässt sich eine Entzündung eines Weichgewebes, wie z. B. einer Gingiva oder einer Mundschleimhaut, zuverlässig identifizieren. Es ist vorstellbar, dass eine räumliche Auflösung der Magnetresonanzmessung bei der Bestimmung einer Entzündung aufgrund des hohen Weichgewebekontrasts der Magnetresonanzmessung reduziert werden kann. Eine räumliche Auflösung ist typischerweise durch eine Anzahl von Bildelementen eines Bildabschnitts des Magnetresonanzbilds definiert. Eine reduzierte räumliche Auflösung kann beispielsweise vorliegen, wenn eine Abmessung des Bildelements einer Abmessung von einigen hundert Mikrometern bis zu wenigen Millimetern einer abgebildeten Anatomie entspricht. Die Bestimmung einer Entzündung kann insbesondere im Rahmen einer Planung und/oder Nachsorge eines chirurgischen Eingriffs, wie z. B. einer Zahnwurzelbehandlung, erfolgen. Ferner kann die Durchführung der Analyse der Abschnitte des Gebisses auch ein Bestimmen einer Zahnkaries und/oder einer Zyste in einer Umgebung der Anzahl von Zähnen umfassen. Es ist vorstellbar, dass die Bestimmung der Zyste an einem Zahn oder einem Kieferbereich des Untersuchungsobjekts ebenfalls anhand von Magnetresonanzdaten und/oder Magnetresonanzbildern mit einem hohen Weichgewebekontrast erfolgt. Dagegen lässt sich eine Zahnkaries zuverlässig anhand eines Kontrastunterschieds zu dem Zahnschmelz oder dem Dentin eines Zahns identifizieren. Der Kontrastunterschied kann sich beispielsweise infolge einer unterschiedlichen Relaxationszeit eines von Zahnkaries betroffenen Abschnitts gegenüber einem gesunden Zahnschmelz oder Dentin ergeben. Für die Bestimmung einer Zahnkaries kann die Magnetresonanzmessung somit einen Bildgebungsparameter und/oder eine Bildgebungssequenz aufweisen, welche üblicherweise für die Bildgebung sehr fester Gewebe verwendet werden. Neben der Entzündung und der Zahnkaries lässt sich selbstverständlich eine Vielzahl weiterer Auffälligkeiten mittels der Analyse der Abschnitte des Gebisses bestimmen. Mögliche Beispiele für Auffälligkeiten sind eine Fehlstellung eines Zahns, eine Verschiebung eines Zahns im Rahmen einer kieferorthopädischen Maßnahme, eine Parodontose, eine Beschädigung und/oder Fraktur eines Zahns, eine Progression eines Weisheitszahns in einem Kieferbereich und dergleichen.

Aufgrund des hohen Weichgewebekontrasts der Magnetresonanzmessung lässt sich das Vorliegen einer Entzündung und/oder einer Zyste schnell und zuverlässig bestimmen. Dadurch kann bei einer Nachsorge einer Zahnbehandlung, wie z. B. einer Zahnwurzelbehandlung, rechtzeitig eine Behandlung eingeleitet werden. Insbesondere bei der Identifikation von Entzündungen eines Weichgewebes des Gebisses kann die Erfassung der Magnetresonanzdaten vorteilhaft mit einer groben Auflösung erfolgen, wodurch sich eine notwendige Zeitdauer für die Durchführung der Magnetresonanzmessung reduzieren lässt.

In einer Ausführungsform umfasst das erfindungsgemäße Verfahren einen weiteren Schritt, in welchem eine Relativposition zwischen einer Auffälligkeit des zumindest einen Abschnitts und zumindest einem Zahn, welcher den zumindest einen Abschnitt aufweist, bestimmt wird, wobei das Erstellen der Übersichtskarte in Abhängigkeit der Relativposition zwischen der Auffälligkeit des zumindest einen Abschnitts und dem zumindest einen Zahn erfolgt. Der zumindest eine Abschnitt mit der Auffälligkeit kann dabei an einem Zahn vorliegen oder sich über eine Mehrzahl von Zähnen erstrecken. Die Bestimmung der Relativposition zwischen der Auffälligkeit des zumindest einen Abschnitts und dem zumindest einen Zahn kann beispielsweise anhand von Lageinformationen der Auffälligkeit und des zumindest einen Zahns erfolgen. Eine Lageinformation kann eine Koordinate oder eine Mehrzahl von Koordinaten in einem von dem Bildgebungsvolumen definierten Koordinatensystem umfassen. In einem einfachen Beispiel stellt eine erste Koordinate einen Flächenschwerpunkt oder einen Volumenschwerpunkt der Auffälligkeit dar, während eine zweite Koordinate einen Flächenschwerpunkt oder einen Volumenschwerpunkt des zumindest einen Zahns darstellt. Die Relativposition zwischen der Auffälligkeit des zumindest einen Abschnitts und dem zumindest einen Zahn kann mittels Korrelation der ersten Koordinaten und der zweiten Koordinate in dem Bildgebungsvolumen bestimmt werden. Es ist ebenso vorstellbar, dass die Lageinformation Koordinaten einer Mehrzahl von Punkten aufweist, welche beispielsweise entlang einer Kontur der Auffälligkeit und/oder einer Kontur des zumindest einen Zahns verteilt sind. Die Mehrzahl von Punkten können eine Fläche, ein Volumen und/oder eine Position der Auffälligkeit und des zumindest einen Zahns in dem Bildgebungsvolumen definieren, welche zu der Bestimmung der Relativposition zwischen der Auffälligkeit des zumindest einen Abschnitts und dem zumindest einen Zahn herangezogen werden können.

Vorzugsweise werden die Lageinformationen der Auffälligkeit und/oder des zumindest einen Zahns anhand von Kontrasten des Magnetresonanzbilds oder von Signalintensitätswerten der Magnetresonanzdaten bestimmt. Eine räumliche Zuordnung einzelner Bildelemente des Magnetresonanzbilds zu dem Bildgebungsvolumen kann beispielsweise anhand einer Frequenzkodierung, einer Phasenkodierung sowie einer räumlichen Kodierung erfolgen, welche durch Anlegen von Gradientenfeldern bei der Durchführung der Magnetresonanzmessung erhalten werden. Die Bestimmung der Lageinformationen erfolgt vorzugsweise mittels einer Bildverarbeitungseinheit. Die Bildverarbeitungseinheit kann ferner dazu ausgebildet sein, die Relativposition zwischen der Auffälligkeit und dem zumindest einen Zahn anhand der Lageinformationen der Auffälligkeit und des zumindest einen Zahns zu bestimmen.

Die Erstellung der Gebissübersichtskarte erfolgt in Abhängigkeit der Relativposition zwischen der Auffälligkeit des zumindest einen Abschnitts und dem zumindest einen Zahn. Beispielsweise wird eine Repräsentation der Auffälligkeit bei dem Erstellen der Gebissübersichtskarte relativ zu der Repräsentation des zumindest einen Zahns positioniert, sodass eine Position der Repräsentation der Auffälligkeit zumindest näherungsweise mit einer anatomisch korrekten Position der Auffälligkeit an dem zumindest einen Zahn übereinstimmt.

Durch die Bestimmung der Relativposition zwischen der Auffälligkeit eines Abschnitts und dem zumindest einen Zahn des Abschnitts lässt sich eine Repräsentation der Auffälligkeit auf vorteilhafte Weise in einer annähernd korrekten Lage zu der Repräsentation der Anzahl von Zähnen mittels der Gebissübersichtskarte darstellen. Die Verwendung einer entsprechenden Gebissübersichtskarte kann das Risiko einer Fehldiagnose gegenüber einer Verwendung eines Magnetresonanzbilds auf vorteilhafte Weise reduzieren, da ein Kontrastunterschied zwischen einem gesunden und einem erkrankten Zahn in den Magnetresonanzbildern, insbesondere bei einer Zahnerkrankung im Anfangsstadium, gering ausfallen kann.

In einer weiteren Ausführungsform des erfindungsgemäßen Verfahrens ist die Magnetresonanzmessung eine erste Magnetresonanzmessung, welche zur Erfassung von ersten Magnetresonanzdaten zu einem ersten Zeitpunkt durchgeführt wird, wobei ein erstes Bildgebungsvolumen der ersten Magnetresonanzmessung mit einem ersten Volumen des Gebisses abgestimmt ist und eine erste Anzahl von Zähnen umfasst und wobei der zumindest eine Abschnitt, in welchem die Auffälligkeit bestimmt wird, ein erster Abschnitt ist. Ein erster Zeitpunkt stellt vorzugsweise einen Beginn einer diagnostischen Bildgebung des Gebisses des Untersuchungsobjekts mittels des erfindungsgemäßen Verfahrens dar. Hierfür wird eine erste Magnetresonanzmessung mit einem ersten Bildgebungsvolumen durchgeführt, welches mit dem ersten Volumen des Gebisses abgestimmt ist. Die erste Anzahl von Zähnen des ersten Volumens des Gebisses kann dabei einen diagnostisch relevanten Bereich, wie **z. B.** einen Zahn, zwei Zähne sowie eine Mehrzahl von Zähnen eines Zahnbogens oder beider Zahnbögen des Untersuchungsobjekts, umfassen. Der diagnostisch relevante Bereich kann sich beispielsweise aus einem Verdacht einer vorliegenden Zahnerkrankung und/oder einer visuellen Beurteilung des Zustands des Gebisses durch den behandelnden Mediziner ergeben.

Die erste Magnetresonanzmessung weist insbesondere eine erste Bildgebungssequenz mit ersten Bildgebungsparametern zur Erfassung von ersten Magnetresonanzdaten auf. Die erste Bildgebungssequenz kann eine erste Aufnahmequalität, wie z. B. eine räumliche Auflösung der ersten Magnetresonanzdaten, bestimmen. Es ist vorstellbar, dass die erste Aufnahmequalität in Abhängigkeit eines Verdachts einer vorliegenden Zahnerkrankung und/oder des diagnostisch relevanten Bereichs eingestellt wird. Eine Einstellung der ersten Aufnahmequalität kann z. B. mittels Anpassung einer Schichtdicke und/oder einer Vollständigkeit einer Aufnahme von ersten Magnetresonanzdaten (k-Raum-Daten) erfolgen.

Vorzugsweise werden die ersten Bildgebungsparameter der ersten Bildgebungssequenz zusammen mit den ersten Magnetresonanzdaten und der Gebissübersichtskarte in einer Datenbank abgespeichert. Diese Informationen können bei nachfolgenden Untersuchungen, beispielsweise im Rahmen einer sogenannten longitudinalen Bildgebungsstudie des Gebisses, als Referenz herangezogen werden. Es ist vorstellbar, dass die erste Magnetresonanzmessung eine besonders hohe Auflösung, eine besonders hohe Aufnahmequalität, eine hohe Anzahl verschiedener Gewebekontraste und/oder ein besonders großes Bildgebungsvolumen aufweist, um Magnetresonanzdaten aller Zähne des Untersuchungsobjekts zu erfassen und eine Grundlage für eine quantitative und qualitative Beurteilung des Zustands des Gebisses bereitzustellen. Eine quantitative Beurteilung kann dabei eine Beurteilung des Ausmaßes einer Zahnerkrankung darstellen, während eine qualitative Beurteilung eine Diagnose eines Typs einer vorliegenden Zahnerkrankung umfasst.

In einem weiteren Schritt des erfindungsgemäßen Verfahrens wird eine zweite Magnetresonanzmessung zur Erfassung von zweiten Magnetresonanzdaten des Gebisses zu einem zweiten Zeitpunkt durchgeführt, wobei ein zweites Bildgebungsvolumen der zweiten Magnetresonanzmessung mit einem zweiten Volumen des Gebisses abgestimmt ist und zumindest einen Zahn umfasst und wobei zumindest ein Bildgebungsparameter der zweiten Magnetresonanzmessung in Abhängigkeit der Auffälligkeit des ersten Abschnitts bestimmt wird. Der zweite Zeitpunkt kann sich an den ersten Zeitpunkt in direkter Weise anschließen. Dies kann bedeuten, dass die zweite Magnetresonanzmessung im Anschluss an die erste Magnetresonanzmessung durchgeführt wird. Die Differenz zwischen dem ersten Zeitpunkt und dem zweiten Zeitpunkt kann somit näherungsweise der Zeitdauer der ersten Magnetresonanzmessung entsprechen. Der zweite Zeitpunkt kann aber auch einen größeren Zeitabstand zum ersten Zeitpunkt aufweisen. Beispielsweise kann die zweite Magnetresonanzmessung eine Nachuntersuchung eines Patienten darstellen, wobei der erste Zeitpunkt und der zweite Zeitpunkt mindestens einen Tag, mehr als eine Woche, mehr als einen Monat, mehr als sechs Monate, neun bis 15 Monate oder mehr als 15 Monate auseinanderliegen. Vorzugsweise repräsentieren die erste Magnetresonanzmessung und die zweite Magnetresonanzmessung eine longitudinale Bildgebungsstudie oder einen Teil einer longitudinalen Bildgebungsstudie des Gebisses eines Patienten. Es ist insbesondere vorstellbar, dass sich an die zweite Magnetresonanzmessung eine dritte Magnetresonanzmessung zu einem dritten Zeitpunkt anschließt. Selbstverständlich sind auch weitere Magnetresonanzmessungen zu weiteren Zeitpunkten vorstellbar. Es ist vorstellbar, dass Bildgebungsuntersuchungen zu weiteren Zeitpunkten mit einem unterschiedlichen Bildgebungsverfahren durchgeführt werden. Mögliche Bildgebungsverfahren stellen beispielsweise Röntgen-Projektionsverfahren, hochauflösende Röntgen-Computertomographieverfahren, intraorale Kameras und und/oder Magnetresonanzverfahren mit verschiedenen Scanner-Architekturen dar. Es ist weiterhin vorstellbar, dass die Messungen zu weiteren Zeitpunkten mit der gleichen Magnetresonanzvorrichtung durchgeführt werden, beispielsweise mit einer konventionellen, radiologischen Magnetresonanzvorrichtung oder mit einer dedizierten Magnetresonanzvorrichtung, welche dazu ausgebildet ist, Magnetresonanzdaten des Gebisses eines Patienten zu erfassen. Vorzugsweise wird eine Menge von Messdaten nachfolgender Bildgebungsuntersuchungen reduziert, um eine zeiteffiziente Bildgebung zu ermöglichen. Beispielsweise können das Bildgebungsvolumen, die Aufnahmequalität, die Anzahl von Gewebekontrasten und/oder die Vollständigkeit der Magnetresonanzdaten weiterer Magnetresonanzmessungen in Abhängigkeit von Messdaten und/oder identifizierter Auffälligkeiten vorhergehender Bildgebungsuntersuchungen reduziert werden, um die Zeitdauer von nachfolgenden Bildgebungsuntersuchungen zu verringern.

Bei der Durchführung der zweiten Magnetresonanzmessung wird zumindest ein Bildgebungsparameter in Abhängigkeit der Auffälligkeit des ersten Abschnitts bestimmt. Es ist vorstellbar, dass eine Position und/oder eine Abmessung des zweiten Bildgebungsvolumens gegenüber dem ersten Bildgebungsvolumen verändert wird, um das zweite Bildgebungsvolumen an ein Volumen des Gebisses mit dem ersten Abschnitt anzupassen. Dabei kann ein Zahn oder eine Mehrzahl von Zähnen aus einem Randbereich des ersten Bildgebungsvolumens in das zweite Bildgebungsvolumen aufgenommen oder aus dem zweiten Bildgebungsvolumen ausgeschlossen werden. Es ist ebenso vorstellbar, dass eine zweite Aufnahmequalität der zweiten Magnetresonanzmessung in Abhängigkeit der Auffälligkeit des ersten Abschnitts angepasst wird. Wie oben beschrieben, kann die zweite Aufnahmequalität bei dem Vorliegen einer Entzündung in dem ersten Abschnitt reduziert werden, da ein hoher Weichgewebekontrast der zweiten Magnetresonanzmessung eine zuverlässige Quantifizierung der Entzündung auch bei einer niedrigen Aufnahmequalität ermöglicht. Es ist aber ebenso vorstellbar, dass die zweite Aufnahmequalität gegenüber der ersten Aufnahmequalität erhöht wird, während eine Abmessung des zweiten Bildgebungsvolumens gegenüber dem ersten Bildgebungsvolumen reduziert wird. Dadurch lässt sich ein diagnostisch relevanter Bereich mit einer hohen Auflösung darstellen, während die Zeitdauer der zweiten Magnetresonanzmessung reduziert wird.

In einer Ausführungsform wird eine erste Gebissübersichtskarte in Abhängigkeit der ersten Magnetresonanzdaten erstellt und eine zweite Gebissübersichtskarte anhand der zweiten Magnetresonanzdaten erstellt. Es ist jedoch ebenso vorstellbar, dass eine kombinierte Gebissübersichtskarte in Abhängigkeit der ersten Magnetresonanzdaten und der zweiten Magnetresonanzdaten erstellt wird. Die kombinierte Gebissübersichtskarte kann dabei den Zustand des Gebisses, wie z. B. eine Auffälligkeit des ersten Abschnitts, zu dem ersten Zeitpunkt und dem zweiten Zeitpunkt gegenüberstellen.

Anhand der ersten Magnetresonanzdaten der ersten Magnetresonanzmessung lassen sich Auffälligkeiten des Gebisses des Untersuchungsobjekts auf vorteilhafte Weise dokumentieren. Diese Informationen können im Rahmen einer kontinuierlichen Nachsorge, insbesondere einer longitudinalen Bildgebungsstudie, verwendet werden, um Bildgebungsparameter nachfolgender Bildgebungsuntersuchungen anzupassen und eine Effizienz und/oder eine Qualität der nachfolgenden Bildgebungsuntersuchungen auf vorteilhafte Weise zu erhöhen. Durch die mehrfache Erfassung von Magnetresonanzdaten zu unterschiedlichen Zeitpunkten kann auf vorteilhafte Weise eine systematische, quantitative Beurteilung des Zustands des Gebisses des Untersuchungsobjekts ermöglicht werden. Beispiele für eine vorteilhafte Anwendung einer longitudinalen Bildgebungsstudie mittels des erfindungsgemäßen Verfahrens sind eine morphometrische Analyse der Gingiva zur Beurteilung einer Parodontitis sowie eine Dokumentation einer Progression einer Zahnerkrankung und/oder einer Entwicklung einer Zahnposition im Rahmen einer kieferorthopädischen Maßnahme.

In einer möglichen Ausführungsform des erfindungsgemäßen Verfahrens erfolgt das Durchführen der zweiten Magnetresonanzmessung für eine Erfassung von zweiten Magnetresonanzdaten des ersten Abschnitts mit einer zweiten Aufnahmequalität, wobei die zweite Aufnahmequalität höher oder gleich einer ersten Aufnahmequalität ist, welche bei dem Durchführen der ersten Magnetresonanzmessung für eine Erfassung der ersten Magnetresonanzdaten des ersten Abschnitts eingesetzt wird.

Es ist vorstellbar, dass die zweite Aufnahmequalität gegenüber der ersten Aufnahmequalität erhöht wird, indem die aufzulösende Schichtdicke der zweiten Magnetresonanzmessung gegenüber der ersten Magnetresonanzmessung reduziert wird. Dadurch können die Anzahl der Volumenelemente und die räumliche Auflösung der zweiten Magnetresonanzmessung gegenüber der ersten Magnetresonanzmessung erhöht werden. Vorzugsweise wird die zweite Aufnahmequalität der zweiten Magnetresonanzmessung dabei in Abhängigkeit der Auffälligkeit des ersten Abschnitts der ersten Magnetresonanzmessung bestimmt. Die zweite Aufnahmequalität wird dabei insbesondere so bestimmt, dass die räumliche Auflösung und/oder ein Signal-Rausch-Verhältnis der zweiten Magnetresonanzdaten eine ausreichende Sensitivität für eine zuverlässige Beurteilung der Entwicklung der Auffälligkeit aufweist. Es ist vorstellbar, dass die zweite Aufnahmequalität automatisch von einer Bildverarbeitungseinheit der Magnetresonanzvorrichtung bestimmt wird oder von einem Nutzer der Magnetresonanzvorrichtung, wie z. B. dem behandelnden Mediziner, mittels einer Eingabeeinheit eingegeben wird.

Durch die Erhöhung der zweiten Aufnahmequalität gegenüber der ersten Aufnahmequalität lässt sich ein Detailgrad einer Auffälligkeit auf vorteilhafte Weise erhöhen. Dadurch kann die Progression einer Zahnerkrankung des Untersuchungsobjekts mit einer höheren Genauigkeit quantifiziert werden, wodurch ein Risiko einer Fehlbeurteilung eines Ausmaßes der Zahnerkrankung reduziert wird. Ferner ermöglicht die höhere Aufnahmequalität auf vorteilhafte Weise eine Ableitung von angepassten Maßnahmen zur Therapie einer vorliegenden Zahnerkrankung.

In einer möglichen Ausführungsform des erfindungsgemäßen Verfahrens erfolgt das Durchführen der zweiten Magnetresonanzsequenz für eine Erfassung von zweiten Magnetresonanzdaten des zweiten Abschnitts mit einer dritten Aufnahmequalität, wobei die dritte Aufnahmequalität niedriger ist als eine erste Aufnahmequalität, welche bei dem Durchführen der ersten Magnetresonanzmessung für eine Erfassung der ersten Magnetresonanzdaten des ersten Abschnitts eingesetzt wird. Wie oben beschrieben, kann der zweite Abschnitt eine Teilmenge der Anzahl von Zähnen darstellen, für welche eine Auffälligkeit ausgeschlossen wurde. Der zweite Abschnitt kann daher für eine Beurteilung der Progression einer Zahnerkrankung des ersten Abschnitts vernachlässigbar sein. Es ist jedoch vorstellbar, dass sich in dem zweiten Abschnitt des Gebisses des Untersuchungsobjekts zwischen dem ersten Zeitpunkt und dem zweiten Zeitpunkt eine Zahnerkrankung entwickelt hat. Um eine solche Zahnerkrankung auszuschließen, kann das zweite Bildgebungsvolumen der zweiten Magnetresonanzmessung auch ein Volumen des Gebisses mit dem zweiten Abschnitt umfassen. Die Erfassung von zweiten Magnetresonanzdaten des zweiten Abschnitts erfolgt dabei mit einer dritten Aufnahmequalität, welche gegenüber der ersten Aufnahmequalität reduziert ist. Es ist vorstellbar, dass die dritte Aufnahmequalität selektiv, d. h. lediglich für ein Volumen des Gebisses mit dem zweiten Abschnitt, reduziert wird. Hierbei kann beispielsweise eine dedizierte Bildgebungssequenz verwendet werden, welche eine gröbere räumliche Auflösung aufweist. Eine bevorzugte Möglichkeit zur Reduzierung der dritten Aufnahmequalität gegenüber der zweiten Aufnahmequalität stellt die Erhöhung der aufzulösenden Schichtdicke dar. Eine weitere Möglichkeit zur Reduktion der dritten Aufnahmequalität gegenüber der ersten Aufnahmequalität stellt insbesondere eine niedrigabgetastete Aufnahme von Kernspinresonanzen (k-Raum-Daten) aus dem zweiten Abschnitt des Volumens des Gebisses dar.

Es ist weiterhin vorstellbar, dass auch die zweiten Magnetresonanzdaten des ersten Abschnitts gegenüber der ersten Magnetresonanzmessung mit einer reduzierten Aufnahmequalität erfasst werden, sodass die zweite Aufnahmequalität gegenüber der ersten Aufnahmequalität reduziert ist. Beispielsweise kann anhand der ersten Magnetresonanzdaten mit einer höheren räumlichen Auflösung ein anatomisches Kompartment-Modell erstellt werden, welches eine Rekonstruktion von niedrigabgetasteten, niedrigaufgelösten und/oder mit einem geringeren Bildgebungsvolumen erfassten zweiten Magnetresonanzdaten und/oder zweiten Magnetresonanzbildern ermöglicht. Ferner ist auch ein Einsatz von künstlicher Intelligenz, wie **z. B.** trainierten neuronalen Netzen oder Deep Learning Verfahren, zur Rekonstruktion von niedrigaufgelösten und/oder niedrigabgetasteten zweiten Magnetresonanzdaten und/oder zweiten Magnetresonanzbildern vorstellbar.

Durch die Reduktion der dritten Aufnahmequalität gegenüber der ersten Aufnahmequalität bei der Erfassung von zweiten Magnetresonanzdaten des zweiten Abschnitts lässt sich eine Zeitdauer für die Durchführung der zweiten Magnetresonanzmessung auf vorteilhafte Weise reduzieren. Dies kann insbesondere bei pädiatrischen Patienten von Vorteil sein, denen ein Verbleiben in einer Ruheposition bei längeren Messzeiträumen typischerweise schwerfällt.

In einer möglichen Ausführungsform des erfindungsgemäßen Verfahrens erfolgt das Durchführen der zweiten Magnetresonanzmessung für die Erfassung von zweiten Magnetresonanzdaten des ersten Abschnitts mit einer zweiten Aufnahmequalität und das Durchführen der zweiten Magnetresonanzmessung für die Erfassung von zweiten Magnetresonanzdaten des zweiten Abschnitts mit einer dritten Aufnahmequalität, wobei die zweite Aufnahmequalität höher ist als die dritte Aufnahmequalität. In dieser Ausführungsform wird die zweite Magnetresonanzmessung des ersten Abschnitts und des zweiten Abschnitts jeweils mit unterschiedlichen Bildgebungsparametern oder Bildgebungssequenzen durchgeführt, um unterschiedliche Aufnahmequalitäten der zweiten Magnetresonanzdaten des ersten Abschnitts und zweite Magnetresonanzdaten des zweiten Abschnitts zu erfassen. Vorzugsweise werden die Schichtdicke und/oder die Menge der erfassten Kernspinresonanzen dabei so angepasst, dass die zweite Aufnahmequalität des ersten Abschnitts höher ist als die dritte Aufnahmequalität des zweiten Abschnitts.

Durch eine selektive Anpassung der Aufnahmequalitäten unterschiedlicher Abschnitte lässt sich eine Zeiteffizienz der zweiten Magnetresonanzmessung auf vorteilhafte Weise erhöhen, indem zweite Magnetresonanzdaten von Abschnitten mit Auffälligkeiten mit einer erhöhten Auflösung erfasst werden, während Abschnitte, bei denen eine Auffälligkeit bei der ersten Magnetresonanzmessung ausgeschlossen wurde, mit einer geringeren Auflösung erfasst werden.

In einer weiteren Ausführungsform des erfindungsgemäßen Verfahrens ist das zweite Bildgebungsvolumen der zweiten Magnetresonanzmessung auf den ersten Abschnitt des Gebisses begrenzt. Wie oben beschrieben, kann das zweite Bildgebungsvolumen in Abhängigkeit der ersten Magnetresonanzdaten und einer Lageinformation der Auffälligkeit bestimmt werden. Eine Abmessung des zweiten Bildgebungsvolumens kann dabei insbesondere gegenüber einer Abmessung des ersten Bildgebungsvolumens reduziert werden, um eine angepasste Abdeckung des ersten Abschnitts des Gebisses zu erhalten. Es ist ebenso vorstellbar, dass eine Position des zweiten Bildgebungsvolumens relativ zu einer Position des ersten Bildgebungsvolumens verändert wird. Das zweite Bildgebungsvolumen kann dabei auf ein Volumen des Gebisses ausgerichtet werden, welches den ersten Abschnitt mit der Auffälligkeit aufweist. Vorzugsweise werden der zweite Abschnitt und/oder weitere Abschnitte ohne Auffälligkeiten aus dem zweiten Bildgebungsbereich der zweiten Magnetresonanzmessung ausgenommen.

Eine selektive Erfassung von Kernspinresonanzen des ersten Abschnitts des Gebisses kann z. B. mittels eines Einsatzes einer Empfangsantenne erfolgen, welche lokal an dem ersten Abschnitt des Gebisses positioniert wird. Es ist weiterhin vorstellbar, dass die k-Raum-Daten der zweiten Magnetresonanzmessung Kernspinresonanzen eines größeren Bildgebungsvolumens umfassen, die Extraktion von zweiten Magnetresonanzdaten aber auf den ersten Abschnitt des Gebisses begrenzt wird. Eine selektive Extraktion der zweiten Magnetresonanzdaten aus den k-Raum-Daten kann beispielsweise anhand einer Frequenzkodierung, einer Phasenkodierung und/oder einer räumlichen Kodierung erfolgen, mittels derer die Kernspinresonanzen Positionen oder Koordinaten in dem Bildgebungsvolumen zugeordnet werden können. Eine weitere Option stellt die Verwendung eines Kompartment-Modells oder einer Kompartment-Information dar, welche eine optimale k-Raum Abdeckung des ersten Abschnitts bei der Durchführung der zweiten Magnetresonanzmessung ermöglichen.

Durch die Extraktion der zweiten Magnetresonanzdaten des ersten Abschnitts des Gebisses aus den k-Raum-Daten der zweiten Magnetresonanzmessung kann ein Aufwand der Rekonstruktion von zweiten Magnetresonanzbildern auf vorteilhafte reduziert werden. Gleichermaßen lässt sich ein Umfang der erfassten k-Raum-Daten der zweiten Magnetresonanzmessung mittels Verwendung einer lokalen Empfangsantenne zur Erfassung von Kernspinresonanzen des ersten Abschnitts auf vorteilhafte Weise reduzieren. Dadurch können ein Aufwand und/oder eine Zeitdauer für die Rekonstruktion der zweiten Magnetresonanzbilder auf vorteilhafte Weise reduziert werden.

Gemäß einer weiteren Ausführungsform des erfindungsgemäßen Verfahrens erfolgt das Durchführen der zweiten Magnetresonanzmessung mit einer zweiten Bildgebungssequenz, wobei die zweite Bildgebungssequenz in Abhängigkeit der Auffälligkeit des ersten Abschnitts bestimmt wird. Die zweite Bildgebungssequenz kann beispielsweise in Abhängigkeit der Auffälligkeit des ersten Abschnitts, wie z. B. der Lageinformation der Auffälligkeit und/oder des Typs der Zahnerkrankung, bestimmt werden. In einem Beispiel umfasst das Bestimmen der zweiten Bildgebungssequenz eine Anpassung des zweiten Bildgebungsvolumens an den ersten Abschnitt des Gebisses. In einem weiteren Beispiel umfasst das Bestimmen der zweiten Bildgebungssequenz eine Erhöhung der räumlichen Auflösung der zweiten Magnetresonanzmessung gegenüber der ersten Magnetresonanzmessung. Die zweite Bildgebungssequenz kann sich von der ersten Bildgebungssequenz in zumindest einem Bildgebungsparameter, wie z. B. der Schichtdicke, einer Abmessung des Bildgebungsvolumens, einer Echozeit, einer Repititionszeit, einer Abtastungsdichte der Kernspinresonanzen oder dergleichen, unterscheiden. Bildgebungssequenzen für die Darstellung von Zähnen können sehr kurze Echozeiten aufweisen, um eine kurze T2-Relaxationszeit von Spins des Dentins oder des Zahnschmelzes der Zähne zu kompensieren und einen Kontrast oder eine Signalintensität des Dentins oder des Zahnschmelzes in den zweiten Magnetresonanzbildern zu erhöhen. In einem Beispiel wird die Echozeit der zweiten Magnetresonanzmessung in Abhängigkeit einer Zahnkaries in dem ersten Abschnitt angepasst, um eine Sensitivität der zweiten Magnetresonanzmessung bezüglich des Dentins des zumindest einen Zahns des ersten Abschnitts zu erhöhen. Die zweite Bildgebungssequenz kann weiterhin dazu ausgebildet sein, einen Kontrast einer Entzündung, eines injizierten Anästhetikums und/oder eines Materials einer Prothese zu erhöhen.

Durch die Anpassung der zweiten Bildgebungssequenz in Abhängigkeit der Auffälligkeit des ersten Abschnitts lässt sich die zweite Magnetresonanzmessung auf vorteilhafte Weise an eine diagnostisch relevante Fragestellung anpassen. Dadurch können die quantitative und qualitative Beurteilung des Zustands des Gebisses erleichtert und ein Risiko einer Fehlbeurteilung auf vorteilhafte Weise reduziert werden.

In einer möglichen Ausführungsform des erfindungsgemäßen Verfahrens werden erste Magnetresonanzbilder anhand der ersten Magnetresonanzdaten erstellt und zweite Magnetresonanzbilder anhand der zweiten Magnetresonanzdaten erstellt, wobei die ersten Magnetresonanzbilder mit den zweiten Magnetresonanzbildern registriert werden. Die ersten Magnetresonanzbilder und die zweiten Magnetresonanzbilder können mittels bekannter Bildrekonstruktionsverfahren erstellt werden. Eine Registrierung der ersten Magnetresonanzbilder mit den zweiten Magnetresonanzbildern kann unter Verwendung beliebiger Bildregistrierungsverfahren erfolgen. Beispiele für solche Bildregistrierungsverfahren sind flächenbasierte und/oder merkmalsbasierte Verfahren auf Basis einer Korrelationsfunktion, einer Korrespondenz von Kontrollpunkten, einer globalen und/oder lokalen Transformation, einer Mustererkennung, einer radialen Basisfunktion, einer Fourier-Transformation oder dergleichen. Das Bildregistrierungsverfahren kann ferner durch den Einsatz von optischen und/oder magnetischen Markern, einer Verwendung von Orientierungspunkten und/oder einer geometrischen Äquivalenz einer Positionierung des Untersuchungsobjekts relativ zu der Magnetresonanzvorrichtung komplementiert werden. Weiterhin kann die Registrierung der ersten Magnetresonanzbilder und der zweiten Magnetresonanzbilder anhand von teilrigiden Körpermodellen, welche z. B. nur eine relative Bewegung zwischen dem Unterkiefer und dem Oberkiefer beschreiben, erfolgen.

Es ist vorstellbar, dass die ersten Magnetresonanzbilder und die zweiten Magnetresonanzbilder oder Ausschnitte der ersten Magnetresonanzbilder und der zweiten Magnetresonanzbilder zusammen mit der Gebissübersichtskarte ausgegeben werden. Die Gebissübersichtskarte kann ferner Repräsentationen der Anzahl von Zähnen des Untersuchungsobjekts sowie Repräsentationen von Auffälligkeiten aufweisen, welche in Abhängigkeit der registrierten ersten Magnetresonanzbilder und der zweiten Magnetresonanzbilder erstellt werden. Weiterhin kann eine Entwicklung einer Zahnerkrankung und/oder einer Therapiemaßnahme anhand der registrierten Magnetresonanzbilder bestimmt und mittels eines Hinweises, einer Kennzeichnung oder einer Repräsentation auf einer Gebissübersichtskarte abgebildet werden.

Durch die Registrierung der ersten Magnetresonanzbilder mit den zweiten Magnetresonanzbildern lässt sich ein Fortschritt und/oder ein Erfolg einer Therapie einer Zahnerkrankung im Rahmen einer longitudinalen Bildgebungsstudie auf vorteilhafte Weise dokumentieren.

In einer möglichen Ausführungsform umfasst das erfindungsgemäße Verfahren einen weiteren Schritt, in welchem eine Abweichung zwischen den ersten Magnetresonanzdaten und den zweiten Magnetresonanzdaten bestimmt wird, wobei eine Information über die Abweichung zusammen mit der Gebissübersichtskarte ausgegeben wird. Eine Abweichung zwischen den ersten Magnetresonanzdaten und den zweiten Magnetresonanzdaten kann beispielsweise anhand einer Differenz von Werten, wie z. B. Kontrastwerten oder Signalintensitätswerten, eines Datenraums der ersten Magnetresonanzdaten und der zweiten Magnetresonanzdaten bestimmt werden. Solche Werte des Datenraums können z. B. in Form von Tupeln, Vektoren und/oder Matrizen vorliegen und einer Position in einem Bildgebungsvolumen zuordenbar sein. Es ist ebenso vorstellbar, dass die Bestimmung der Abweichung anhand von ersten Magnetresonanzbildern und zweiten Magnetresonanzbildern erfolgt, welche aus den ersten Magnetresonanzdaten und den zweiten Magnetresonanzdaten rekonstruiert werden. Dabei können eine Signalintensität oder ein Kontrast von Bildelementen und/oder Volumenelementen der ersten Magnetresonanzbilder und der zweiten Magnetresonanzbilder miteinander korreliert werden, um eine Abweichung zu bestimmen. Die Bestimmung der Abweichung zwischen den ersten Magnetresonanzbildern und den zweiten Magnetresonanzbildern erfolgt vorzugsweise mittels einer Bildverarbeitungseinheit.

Es ist vorstellbar, dass die Gebissübersichtskarte in Abhängigkeit der Abweichung zwischen den ersten Magnetresonanzdaten und den zweiten Magnetresonanzdaten ergänzt oder erweitert wird. Dies kann bedeuten, dass eine Repräsentation eines Zahns und/oder eine anhand der zweiten Magnetresonanzbilder bestimmte Auffälligkeit des ersten Abschnitts der Gebissübersichtskarte hinzugefügt und/oder dieser überlagert wird. Eine Erweiterung der Gebissübersichtskarte kann beispielsweise ein Hinzufügen eines Hinweises umfassen, welcher eine Information über ein Ausmaß und/oder einen Fortschritt einer Zahnerkrankung zwischen dem ersten Zeitpunkt und dem zweiten Zeitpunkt anzeigt. Es ist insbesondere vorstellbar, dass eine Repräsentation einer in Abhängigkeit der Abweichung zwischen den ersten Magnetresonanzdaten und den zweiten Magnetresonanzdaten bestimmten Differenz einer Auffälligkeit auf der Gebissübersichtskarte dargestellt wird. Eine solche Repräsentation kann beispielsweise eine schematisierte Darstellung der Differenz des Volumens, der Fläche und/oder der Position der Auffälligkeit zwischen dem ersten Zeitpunkt und dem zweiten Zeitpunkt umfassen. Es ist ferner vorstellbar, dass die Repräsentation der Differenz der Auffälligkeit in Abhängigkeit einer Lageinformation der Auffälligkeit annähernd in einer anatomisch korrekten Relativposition zu der Repräsentation der Anzahl von Zähnen der Gebissübersichtskarte dargestellt wird. Weiterhin kann die Gebissübersichtskarte auch miteinander registrierte Ausschnitte der ersten Magnetresonanzbilder und der zweiten Magnetresonanzbilder aufweisen, wobei eine Abweichung zwischen den Ausschnitten der ersten Magnetresonanzbilder und den Ausschnitten der zweiten Magnetresonanzbilder vorzugsweise farblich hervorgehoben ist.

Daneben können beliebige weitere Abweichungen des Zustands des Gebisses zwischen dem ersten Zeitpunkt und dem zweiten Zeitpunkt in der kombinierten Gebissübersichtskarte markiert oder hervorgehoben werden. Bei der Dokumentation des Zustands des Gebisses mittels der Gebissübersichtskarte können insbesondere absolute und/oder relative Angaben oder Einheiten verwendet werden. Beispielsweise kann der Hinweis eine relative Reduktion einer Zahnkaries zu einem zweiten Zeitpunkt im Vergleich zu einem ersten Zeitpunkt umfassen. Die Bestimmung der relativen Reduktion der Zahnkaries erfolgt vorzugsweise mittels einer Bildverarbeitungseinheit, welche eine Lageinformation der Auffälligkeit zu einem ersten Zeitpunkt und einem zweiten Zeitpunkt bestimmt und anschließend die relative Reduktion ermittelt. Selbstverständlich lässt sich eine Ausbreitung einer Zahnerkrankung von einem ersten Zeitpunkt zu einem zweiten Zeitpunkt analog bestimmen und mittels der Gebissübersichtskarte ausgeben. Es ist weiterhin vorstellbar, dass die relative Reduktion oder die relative Ausbreitung in Relation zu einem Referenzwert und/oder einem statistischen Normalwertbereich angegeben werden.

Durch die Bestimmung der Abweichung zwischen den ersten Magnetresonanzdaten und den zweiten Magnetresonanzdaten und die Bereitstellung der Abweichung mittels der Gebissübersichtskarte lassen sich eine Entwicklung einer Zahnerkrankung und/oder ein Effekt einer kieferorthopädischen Behandlung zwischen dem ersten Zeitpunkt und dem zweiten Zeitpunkt auf vorteilhafte Weise darstellen und dokumentieren. Dadurch können ein Aufwand einer Diagnose, einer Bereitstellung von Ergebnissen der Magnetresonanzmessung und/oder einer Unterrichtung des Untersuchungsobjekts über ein Ergebnis einer Therapiemaßnahme auf vorteilhafte Weise reduziert werden.

Die erfindungsgemäße Magnetresonanzvorrichtung umfasst eine Recheneinheit, welche dazu ausgelegt ist, ein erfindungsgemäßes Verfahren zu koordinieren und mittels der Magnetresonanzvorrichtung auszuführen. Zur Erfassung, Verarbeitung und Speicherung von Daten, wie **z. B.** der ersten Magnetresonanzdaten, der zweiten Magnetresonanzdaten, der ersten Magnetresonanzbilder, der zweiten Magnetresonanzbildern und der Gebissübersichtskarte, kann die Magnetresonanzvorrichtung neben der Recheneinheit eine Steuereinheit, einen Arbeitsspeicher, einen Datenspeicher sowie eine geeignete Schnittstelle zur Eingabe und Ausgabe von Daten aufweisen. Die Recheneinheit kann beispielsweise einen Kontroller, einen Mikrokontroller, eine CPU, eine GPU oder dergleichen umfassen. Der Arbeitsspeicher und der Datenspeichern können Speichertechnologien wie RAM, ROM, PROM, EPROM, EEPROM, Flash-Speicher, aber auch HDD Speicher, SSD Speicher oder dergleichen aufweisen. Es ist vorstellbar, dass der Datenspeicher eine interne Speichereinheit darstellt, welche elektrisch und/oder mechanisch mit der Recheneinheit der Magnetresonanzvorrichtung verbunden ist. Es ist aber ebenso vorstellbar, dass der Datenspeicher eine externe Speichereinheit ist, welche mittels einer Netzwerkverbindung mit der Recheneinheit verbunden ist. Beispiele für externe Speichereinheiten sind Netzwerk-Server mit entsprechenden Datenspeichern sowie Cloud-Speicher. Die Daten können mittels analoger und/oder digitaler Signale sowie geeigneter elektrischer oder kabelloser Signalverbindungen zwischen den Komponenten der Magnetresonanzvorrichtung übertragen werden.

Die Recheneinheit ist vorzugsweise elektrisch mit einer Steuereinheit der der Magnetresonanzvorrichtung verbunden und/oder in die Steuereinheit integriert. Die Steuereinheit kann dazu ausgelegt sein, unter Koordination der Recheneinheit ein erfindungsgemäßes Verfahren durchzuführen. Die Steuereinheit kann beispielsweise dazu ausgebildet sein, eine Magnetresonanzmessung des Untersuchungsobjekts durchzuführen, Magnetresonanzdaten des Untersuchungsobjekts zu erfassen und die Magnetresonanzdaten an andere Komponenten, wie **z. B.** die Recheneinheit und/oder die Speichereinheit, zu übertragen. Die Recheneinheit kann dazu ausgelegt sein, die Magnetresonanzdaten einzulesen und Magnetresonanzbilder anhand der Magnetresonanzdaten zu erstellen. Weiterhin kann die Recheneinheit dazu ausgelegt sein, eine Analyse von Abschnitten des Gebisses des Untersuchungsobjekts durchzuführen. Die Recheneinheit weist vorzugsweise eine Bildverarbeitungseinheit auf, welche dazu ausgelegt ist, eine Auffälligkeit eines ersten Abschnittes sowie eine Lageinformation der Auffälligkeit zu bestimmen. Ferner können die Recheneinheit und/oder die Bildverarbeitungseinheit dazu ausgebildet sein, eine Gebissübersichtskarte eines Untersuchungsobjekts in Abhängigkeit von Magnetresonanzdaten und/oder Magnetresonanzbildern des Untersuchungsobjekts zu erstellen und an die Speichereinheit zu übertragen. Die Gebissübersichtskarte und/oder die Magnetresonanzbilder des Untersuchungsobjekts können von der Recheneinheit mittels einer Ausgabeschnittstelle an eine Anzeigeeinheit und/oder ein Mobilgerät übertragen werden.

Die Komponenten der erfindungsgemäßen Magnetresonanzvorrichtung können so aufeinander abgestimmt sein, dass eine zeiteffiziente und robuste Durchführung eines erfindungsgemäßen Verfahrens ermöglicht wird. Insbesondere können das erfindungsgemäße Verfahren oder Teilschritte des erfindungsgemäßen Verfahrens automatisch erfolgen, sodass eine Koordination einzelner Verfahrensschritte weitestgehend autark erfolgt. Die Durchführung eines erfindungsgemäßen Verfahrens erfordert somit keine Fachkenntnis und lässt sich von einem beliebigen Mitglied eines medizinischen Personals initiieren.

Das erfindungsgemäße Computerprogrammprodukt ist in einen Datenspeicher einer Recheneinheit der Magnetresonanzvorrichtung ladbar und weist Programmcode-Mittel auf, um ein erfindungsgemäßes Verfahren auszuführen, wenn das Computerprogrammprodukt in der Recheneinheit der Magnetresonanzvorrichtung ausgeführt wird. Durch das erfindungsgemäße Computerprogrammprodukt kann das erfindungsgemäße Verfahren schnell, identisch wiederholbar und robust ausgeführt werden. Das Computerprogrammprodukt ist so konfiguriert, dass es mittels der Recheneinheit die erfindungsgemäßen Verfahrensschritte ausführen kann. Die Recheneinheit muss dabei jeweils die Voraussetzungen, wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk, einem Server oder einer Cloud hinterlegt, von wo es in den Prozessor einer lokalen Recheneinheit geladen werden kann. Die Recheneinheit kann dabei als eine eigenständige Systemkomponente oder als ein Teil der Magnetresonanzvorrichtung ausgebildet sein. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in der Recheneinheit der Magnetresonanzvorrichtung ein erfindungsgemäßes Verfahren durchführen. Beispiele für elektronisch lesbare Datenträger sind eine DVD, ein Magnetband, ein USB-Stick oder beliebige andere Datenspeicher, auf welchen elektronisch lesbare Steuerinformationen, insbesondere Software, gespeichert ist. Wenn diese Steuerinformationen von dem Datenträger gelesen und an eine Steuereinheit und/oder die Recheneinheit der Magnetresonanzvorrichtung übertragen werden, können alle erfindungsgemäßen Ausführungsformen des beschriebenen, erfindungsgemäßen Verfahrens durchgeführt werden.

Weitere Vorteile und Einzelheiten der vorliegenden Erfindung ergeben sich aus den im Folgenden beschriebenen Ausführungsbeispielen sowie anhand der Zeichnungen. Dabei zeigen:
Fig. 1 eine Ausführungsform einer erfindungsgemäßen Magnetresonanzvorrichtung,
Fig. 2 eine schematische Darstellung einer Gebissübersichtskarte gemäß einer ersten Ausführungsform des erfindungsgemäßen Verfahrens,
Fig. 3 eine schematische Darstellung einer Gebissübersichtskarte gemäß einer zweiten Ausführungsform des erfindungsgemäßen Verfahrens,
Fig. 4 eine schematische Darstellung einer Gebissübersichtskarte gemäß einer dritten Ausführungsform des erfindungsgemäßen Verfahrens,
Fig. 5 eine schematische Darstellung einer Gebissübersichtskarte gemäß einer vierten Ausführungsform des erfindungsgemäßen Verfahrens,
Fig. 6 ein mögliches Ablaufschema gemäß einer Ausführungsform des erfindungsgemäßen Verfahrens.

In der nachfolgenden Figurenbeschreibung wird sich auf einen menschlichen Patienten als Untersuchungsobjekt bezogen, da dieser einen üblichen Anwendungsfall für eine Bildgebungsuntersuchung darstellt. Dies schließt eine Anwendung des erfindungsgemäßen Verfahrens an anderen Untersuchungsobjekten selbstverständlich nicht aus.

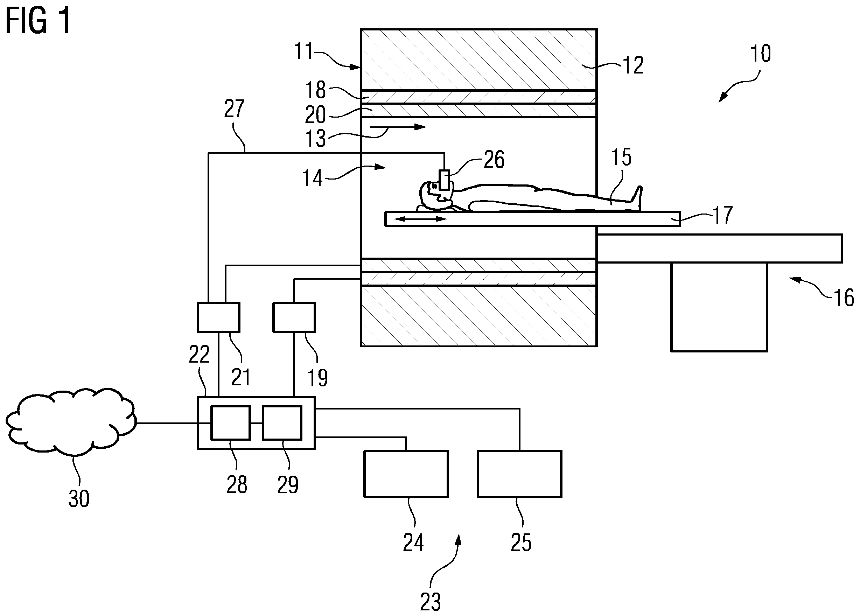

In Fig. 1 ist eine mögliche Ausführungsform der erfindungsgemäßen Magnetresonanzvorrichtung 10 dargestellt. Die Magnetresonanzvorrichtung 10 umfasst eine Magneteinheit 11, welche z. B. einen Permanentmagneten, einen Elektromagneten oder einen supraleitenden Hauptmagneten 12 zur Erzeugung eines starken und insbesondere homogenen Hauptmagnetfelds 13 aufweist. Zudem umfasst die Magnetresonanzvorrichtung 10 einen Patientenaufnahmebereich 14 zu einer Aufnahme eines Patienten. Der Patientenaufnahmebereich 14 ist im vorliegenden Ausführungsbeispiel zylinderförmig ausgebildet und in einer Umfangsrichtung von der Magneteinheit 11 umgeben. Grundsätzlich sind jedoch auch von diesem Beispiel abweichende Ausbildungen des Patientenaufnahmebereichs 14 vorstellbar.

Der Patient kann mittels einer Patientenlagerungsvorrichtung 16 der Magnetresonanzvorrichtung 10 in dem Patientenaufnahmebereich 14 positioniert werden. Die Patientenlagerungsvorrichtung 16 weist hierfür einen innerhalb des Patientenaufnahmebereichs 14 bewegbar ausgestalteten Patiententisch 17 auf. Die Magneteinheit 11 weist weiterhin eine Gradientenspule 18 zum Erzeugen von magnetischen Gradientenfeldern auf, welche für eine Ortskodierung während einer Bildgebung verwendet wird. Die Gradientenspule 18 wird mittels einer Gradientensteuereinheit 19 der Magnetresonanzvorrichtung 10 angesteuert. Die Magneteinheit 11 kann weiterhin eine Hochfrequenzantenne umfassen, welche im vorliegenden Ausführungsbeispiel als fest in die Magnetresonanzvorrichtung 10 integrierte Körperspule 20 ausgebildet ist. Die Körperspule 20 ist zu einer Anregung von Kernspins ausgelegt, die sich in dem von dem Hauptmagneten 12 erzeugten Hauptmagnetfeld 13 befinden. Die Körperspule 20 wird von einer Hochfrequenzeinheit 21 der Magnetresonanzvorrichtung 10 angesteuert und strahlt hochfrequente Anregungsimpulse in eine Bildaufnahmeregion ein, die im Wesentlichen von einem Patientenaufnahmebereich 14 der Magnetresonanzvorrichtung 10 gebildet ist. Die Körperspule 20 ist weiterhin zum Empfang von Kernspinresonanzen ausgebildet.

Zu einer Steuerung des Hauptmagneten 12, der Gradientensteuereinheit 19 und zur Steuerung der Hochfrequenzeinheit 21 weist die Magnetresonanzvorrichtung 10 eine Steuereinheit 22 auf. Die Steuereinheit 22 ist dazu ausgebildet eine Durchführung einer Sequenz, wie z. B. einer bildgebenden GRE (gradient echo) Sequenz, einer TSE (turbo spin echo) Sequenz oder einer UTE (ultra-short echo time) Sequenz, zu steuern. Zudem umfasst die Steuereinheit 22 eine Recheneinheit 28 zu einer Auswertung von Magnetresonanzdaten, die während einer Magnetresonanzmessung erfasst werden. Die Recheneinheit 28 der Magnetresonanzvorrichtung 10 kann dazu ausgebildet sein, Rekonstruktionsmethoden einzusetzen, um Magnetresonanzbilder anhand der Magnetresonanzdaten zu rekonstruieren. Ferner kann die Recheneinheit dazu ausgebildet sein, eine Gebissübersichtskarte 40 in Abhängigkeit der Magnetresonanzdaten zu erstellen. Die Recheneinheit 28 ist im vorliegenden Beispiel mit einer Speichereinheit 29 sowie mit einem Cloud-Speicher 30 verbunden. Die Recheneinheit kann dazu ausgelegt sein, Daten wie z. B. Magnetresonanzbilder, Magnetresonanzdaten und/oder Gebissübersichtskarten 40 auf der Speichereinheit 29 und dem Cloud-Speicher 30 zu speichern und diese Daten von der Speichereinheit oder dem Cloud-Speicher mittels einer geeigneten Schnittstelle abzurufen. Es ist ebenso vorstellbar, dass der Patient 15 mittels einer geeigneten Software-Anwendung von einem Mobilgerät (nicht gezeigt) Zugriff auf einen Speicherbereich erhält, welcher Magnetresonanzbilder und/oder Gebissübersichtskarten des Patienten 15 enthält. Die Software-Anwendung kann entsprechend dazu ausgelegt sein, die Magnetresonanzbilder und/oder die Gebissübersichtskarte 40 auf einem Bildschirm des Mobilgeräts auszugeben.

Des Weiteren umfasst die Magnetresonanzvorrichtung 10 eine Benutzerschnittstelle 23, welche eine Signalverbindung mit der Steuereinheit 22 aufweist. Steuerinformationen, wie z. B. Bildgebungsparameter, aber auch rekonstruierte Magnetresonanzbilder und/oder Gebissübersichtskarten 40, können auf einer Anzeigeeinheit 24, beispielsweise auf zumindest einem Monitor, der Benutzerschnittstelle 23 für einen Nutzer angezeigt werden. Weiterhin weist die Benutzerschnittstelle 23 eine Eingabeeinheit 25 auf, mittels der Parameter einer Magnetresonanzmessung von dem Nutzer eingegeben werden können. Die Magnetresonanzvorrichtung 10 kann ferner eine lokale Empfangsantenne 26 aufweisen, welche an dem Gebiss eines Patienten 15 positioniert ist und Kernspinresonanzen eines Zahns oder einer Mehrzahl von Zähnen des Patienten 15 erfasst und an die Recheneinheit 28 der Steuereinheit 22 überträgt. Die lokale Empfangsantenne 26 weist vorzugsweise eine elektrische Anschlussleitung 27 auf, welche eine Signalverbindung mit der Hochfrequenzeinheit 21 und der Steuereinheit 22 bereitstellt. Ebenso wie die Körperspule 20 kann auch die lokale Empfangsantenne 26 zu einer Anregung von Kernspins und zum Empfang von Kernspinresonanzen ausgebildet sein. Die lokale Empfangsantenne 26 kann hierfür insbesondere einen trommelförmigen Aufbau aufweisen, welcher einen Kopf des Patienten 15 umschließt. Zum Aussenden von hochfrequenten Anregungsimpulsen wird die lokale Empfangsantenne 26 von der Hochfrequenzeinheit 21 angesteuert.

Die dargestellte Magnetresonanzvorrichtung 10 kann selbstverständlich weitere Komponenten umfassen, welche Magnetresonanzvorrichtungen üblicherweise aufweisen. Es ist ebenso vorstellbar, dass die Magnetresonanzvorrichtung 10 statt des zylinderförmigen Aufbaus einen C-förmigen, einen dreieckigen oder einen asymmetrischen Aufbau der Magnetfeld-erzeugenden Komponenten aufweist. Die Magnetresonanzvorrichtung 10 kann insbesondere dazu ausgebildet ein, eine Magnetresonanzuntersuchung eines stehenden oder sitzenden Patienten 15 durchzuführen. Es ist ferner vorstellbar, dass die Magnetresonanzvorrichtung 10 speziell dazu ausgebildet ist, Bildgebungsuntersuchungen des Gebisses eines Patienten 15 durchzuführen.

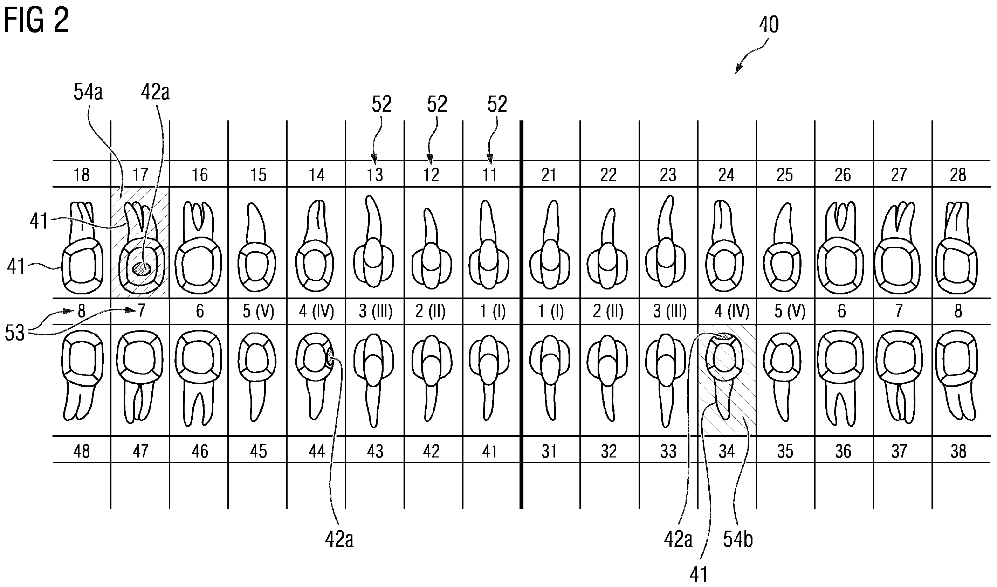

Fig. 2 zeigt eine schematische Darstellung einer Gebissübersichtskarte 40. Die Gebissübersichtskarte 40 umfasst eine geordnete Darstellung der Anzahl von Zähnen des Patienten 15. Einzelne Zähne des Patienten 15 sind dabei als Repräsentationen eines Zahns 41 dargestellt. Die Repräsentationen der Zähne 41 sind dabei so gestaltet, dass sich ein Typ eines Zahns anhand der schematisierten Darstellung der Repräsentation des Zahns 41 identifizieren lässt. Die Gebissübersichtskarte 40 weist ferner Nummerierungen 52 auf, welche jedem Zahn der Anzahl von Zähnen des Gebisses des Patienten eine eindeutige Identifikationsnummer zuweisen. In dem vorliegenden Beispiel werden die Zähne einer linken Seite und einer rechten Seite des Oberkiefers und des Unterkiefers in fortlaufender Weise durchnummeriert, wobei jeweils mit einem Frontschneidezahn begonnen wird. Die Gebissübersichtskarte 40 weist ferner Kennzeichnungen 53 auf, welche eine jeweilige Position eines Zahns entlang der Zahnbögen angibt. Selbstverständlich sind neben dem gezeigten Verfahren zur Nummerierung und/oder Kennzeichnung der Zähne auch beliebige andere Verfahren vorstellbar.

Die Gebissübersichtskarte 40 weist ferner eine Markierung 54a auf, welche eine Entzündung des Zahns mit der Nummer "17" an der siebten Position der linken Seite des Oberkiefers anzeigt. Die Markierung 54a ist in dem gezeigten Beispiel als ein Rahmen ausgeführt, welcher den Zahn mit der Nummer "17" umschließt. Der Zahn mit der Nummer "34" an der vierten Position des rechten Unterkiefers weist dagegen beispielsweise eine Zahnkaries auf. Die Repräsentation der Zahnkaries 42a ist dabei so an der Repräsentation des Zahns 41 positioniert, dass eine Relativposition zwischen der Repräsentation des Zahns 41 und der Repräsentation der Zahnkaries 42b mit einer anatomisch korrekten Relativposition zwischen der Zahnkaries und dem betreffenden Zahn annähernd übereinstimmt. Eine Form und/oder Abmessung der Repräsentation der Zahnkaries 42a kann dabei mit einer Form und/oder einer Abmessung der Zahnkaries korreliert sein. Der Zahn mit der Nummer "34" weist in dem gezeigten Beispiel zusätzlich eine Markierung 54b auf, welche sich farblich von der Markierung 54a unterscheidet und auf das Vorliegen einer Entzündung hinweist. Der Zahn mit der Nummer "44" weist dagegen lediglich eine Repräsentation einer Zahnkaries 42a auf. Eine zusätzliche Zahnentzündung kann daher bei diesem Zahn ausgeschlossen werden.

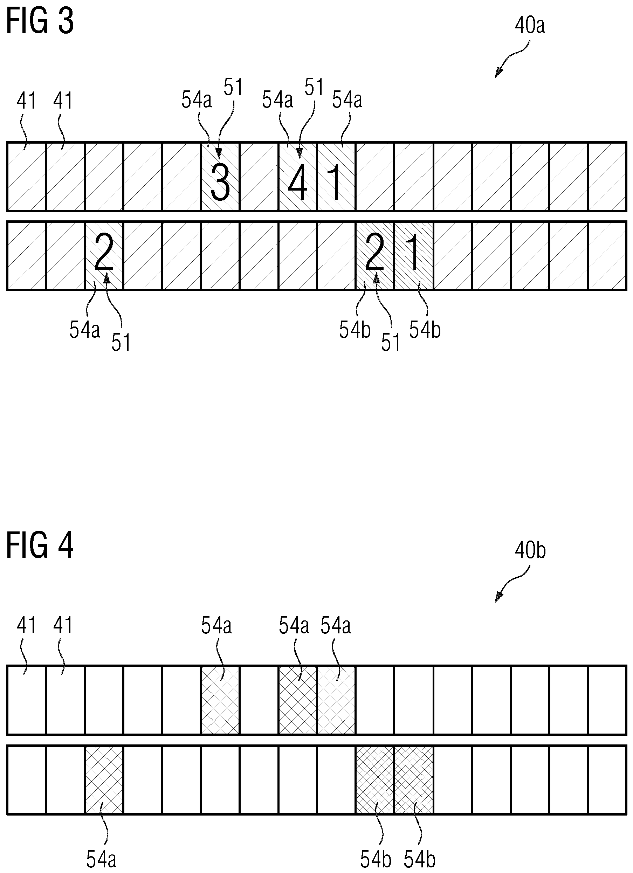

Fig. 3 zeigt eine vereinfachte Darstellungsform der Gebissübersichtskarte 40. In dem vorliegenden Beispiel wurde die Gebissübersichtskarte 40a anhand von ersten Magnetresonanzdaten einer ersten Magnetresonanzmessung erstellt. Die Repräsentation der Zähne 41 erfolgt bei dieser Darstellungsform mittels einfacher Boxen, welche stellvertretend für die beiden Zahnbögen des Gebisses des Patienten 15 in zwei Reihen angeordnet sind. Die Gebissübersichtskarte 40a weist Markierungen 54a und 54b auf, welche das Vorliegen verschiedener Zahnerkrankungen anzeigen. In dem vorliegenden Beispiel zeigt die Markierung 54a eine Zahnentzündung an, während die Markierung 54b eine Zahnkaries anzeigt. Die Markierungen 54a und 54b können sich dabei z. B. in einer Farbe, einer Graustufe und/oder einem Muster unterscheiden, um verschiedene Zahnerkrankungen anzuzeigen. Die Repräsentationen von Zähnen 41 mit den Markierungen 54a und 54b weisen ferner Hinweise 51 auf, welche ein Ausmaß und/oder ein Fortschrittsstadium der jeweils vorliegenden Zahnerkrankung kodieren. In dem gezeigten Beispiel nimmt ein Schweregrad der Zahnerkrankung auf einer Skala von "1" bis "10" zu. Die Ziffer "1" kann beispielsweise bedeuten, dass sich die Zahnerkrankung an dem betreffenden Zahn noch in einem Anfangsstudium befindet. Die Ziffer "4" kann hingegen bedeuten, dass sich die Zahnerkrankung bereits in einem fortgeschrittenen Stadium befindet.

Fig. 4 zeigt eine zweite Gebissübersichtskarte 40b, welche beispielsweise anhand der zweiten Magnetresonanzmessung oder anhand von Messdaten nachfolgender Bildgebungsuntersuchungen erstellt wird. Um die Zeitdauer der zweiten Magnetresonanzmessung zu reduzieren, wurde der zweite Bildgebungsbereich der zweiten Magnetresonanzmessung auf jene Abschnitte beschränkt, welche bei der Analyse der ersten Magnetresonanzdaten eine Auffälligkeit aufgewiesen haben. Ein Fortschritt und/oder ein Ausmaß der Zahnerkrankung ist in dem vorliegenden Beispiel mittels einer Farbe oder einer Graustufe der Markierungen 54a und 54b kodiert. Unterschiedliche Zahnerkrankungen können dabei z. B. anhand von verschiedenen Mustern oder Farben der Markierungen 54a und 54b unterschieden werden. Diese vereinfachte Darstellungsform der zweiten Gebissübersichtskarte 40b ist insbesondere geeignet, an ein Mobilgerät übertragen zu werden und den Patienten 15 über den Zustand des Gebisses zu informieren.

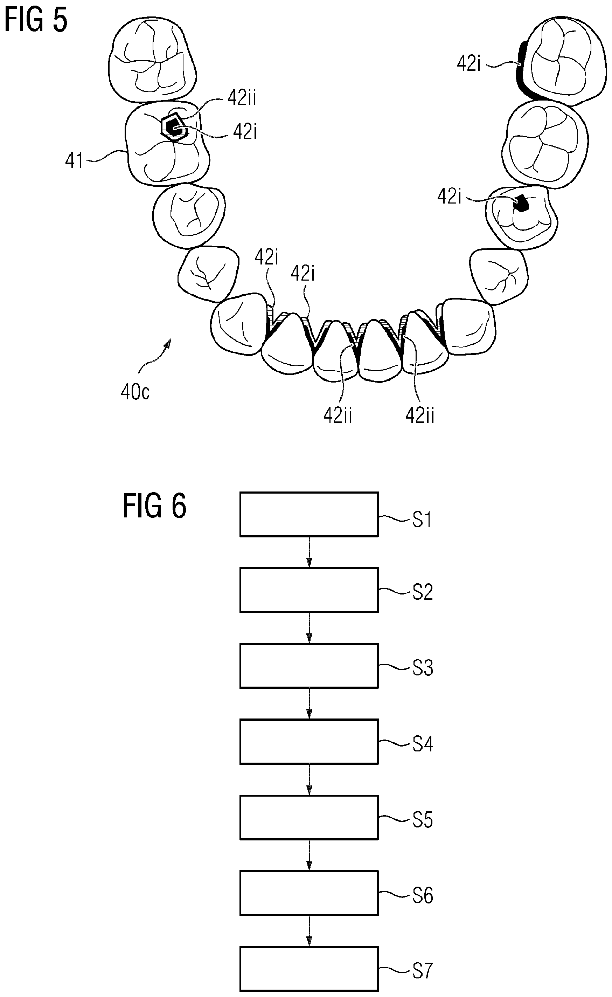

Fig. 5 zeigt eine schematisierte Darstellung einer kombinierten Gebissübersichtskarte 40c, welche in Abhängigkeit der ersten Magnetresonanzdaten und der zweiten Magnetresonanzdaten (und/oder weiterer Messdaten von Bildgebungsuntersuchungen) erstellt wird. In dem dargestellten Beispiel umfasst die kombinierte Gebissübersichtskarte 40c Repräsentationen der Zähne 41 des Unterkiefers des Patienten 15. Vorzugsweise werden die anhand der zweiten Magnetresonanzdaten bestimmten Auffälligkeiten von Abschnitten des Gebisses bei der Erstellung der kombinierten Gebissübersichtskarte 40c einer ersten Gebissübersichtskarte 40a mit einer vergleichbaren Darstellungsform überlagert, sodass eine Progression und/oder eine Entwicklung einer Zahnerkrankung zwischen dem ersten Zeitpunkt und dem zweiten Zeitpunkt anhand der kombinierten Gebissübersichtskarte 40c quantifiziert werden kann. Beispielsweise weist ein Backenzahn des Patienten 15 eine Zahnkaries auf, welche sich zwischen dem ersten Zeitpunkt und dem zweiten Zeitpunkt ausgebreitet hat. Die Ausbreitung der Zahnkaries wird mittels der größeren Abmessung der Repräsentation der Zahnkaries 42ii, welche zu dem zweiten Zeitpunkt bestimmt wird, gegenüber der Repräsentation der Zahnkaries 42i, welche zu dem ersten Zeitpunkt bestimmt wurde, visuell hervorgehoben. In dem vorliegenden Beispiel ist die Ausbreitung der Zahnkaries an weiteren Zähnen des Unterkiefers zwischen dem ersten Zeitpunkt und dem zweiten Zeitpunkt konstant geblieben, weshalb eine Überlagerung mit der Repräsentation der Zahnkaries 42ii der zweiten Gebissübersichtskarte nicht erforderlich ist.

In einem zweiten Beispiel weisen Schneidezähne des Unterkiefers des Patienten 15 in einem Übergangsbereich zur Gingiva eine Parodontitis auf. Das anhand der ersten Magnetresonanzdaten bestimmte Ausmaß des entzündeten Zahnfleischs ist mittels der Repräsentation der Parodontitis 42i in der kombinierten Gebissübersichtskarte 40c dargestellt. Da die Zahnfleischentzündung durch eine entsprechende Therapiemaßnahme zwischen dem ersten Zeitpunkt und dem zweiten Zeitpunkt rückläufig ist, weist der mittels der Repräsentation der Parodontitis 42ii gekennzeichnete Bereich gegenüber der mittels der Repräsentation 42i gekennzeichneten Bereichs eine geringe Fläche auf.

Eine in den Figuren 3, 4 und 5 gezeigte Gebissübersichtskarte 40 wird vorzugsweise anhand von Magnetresonanzdaten einer Magnetresonanzvorrichtung 10 erstellt. Es ist jedoch vorstellbar, dass ein oben beschriebenes, erfindungsgemäßes Verfahren auf andere Bildgebungsverfahren übertragbar ist. Es ist ebenso vorstellbar, dass bestehende Datensätze anderer Bildgebungsverfahren verwendet werden, um eine Gebissübersichtskarte 40 zu erstellen und/oder zu ergänzen, ohne eine erneute Bildgebungsuntersuchung durchzuführen. Mögliche Bildgebungsverfahren können, wie oben beschrieben, z. B. Röntgenverfahren oder Intraoralkameraverfahren darstellen.

Fig. 6 zeigt ein mögliches Ablaufdiagramm eines erfindungsgemäßen Verfahrens zur Erstellung einer Gebissübersichtskarte 40 von einem Gebiss eines Patienten 15 anhand von Magnetresonanzdaten einer Magnetresonanzmessung des Gebisses.

In einem Schritt S1 des erfindungsgemäßen Verfahrens wird eine Magnetresonanzmessung zur Erfassung von Magnetresonanzdaten des Gebisses durchgeführt, wobei ein Bildgebungsvolumen der Magnetresonanzmessung mit einem Volumen des Gebisses abgestimmt ist und wobei das Bildgebungsvolumen eine Anzahl von Zähnen des Gebisses umfasst. Der Patient 15 wird hierfür zunächst in einem Patientenaufnahmebereich 14 positioniert, sodass ein diagnostisch relevanter Bereich des Gebisses mit dem Bildgebungsbereich der Magnetresonanzvorrichtung 10 übereinstimmt. Die Positionierung des Patienten 15 erfolgt vorzugsweise mittels einer Patientenlagerungsvorrichtung 16, welche den Patienten 15 in den zylindrischen Patientenaufnahmebereich 14 einer konventionellen, radiologischen Magnetresonanzvorrichtung 10 transportiert. Es ist aber ebenso vorstellbar, dass die Magnetresonanzvorrichtung 10 eine dedizierte Bildgebungsvorrichtung zur Aufnahme von Magnetresonanzdaten einer Dentalregion des Patienten 15 darstellt, bei welcher der Patientenaufnahmebereich 14 in beliebiger Weise an einen Kopf des Patienten 15 angepasst sein kann. Es beispielsweise vorstellbar, dass die Magnetresonanzvorrichtung 10 entlang einer mechanischen Führung in Relation zu dem Kopf des Patienten 15 positioniert wird, bis das Bildgebungsvolumen mit dem diagnostisch relevanten Bereich des Gebisses übereinstimmt. Der diagnostisch relevante Bereich umfasst dabei eine Anzahl von Zähnen, von denen im Rahmen einer Beurteilung des Zustands des Gebisses Magnetresonanzdaten erfasst werden sollen. Der diagnostisch relevante Bereich kann beispielsweise im Vorfeld der Magnetresonanzmessung mittels eines behandelnden Mediziners festgelegt werden. Das Durchführen der Magnetresonanzmessung kann wie oben beschrieben erfolgen.

In einer Ausführungsform werden multiple Magnetresonanzmessungen des Gebisses des Patienten 15 im Rahmen einer longitudinalen Bildgebungsstudie des Patienten 15 durchgeführt. Hierbei umfasst der Schritt (S1) eine Durchführung einer ersten Magnetresonanzmessung zur Erfassung von ersten Magnetresonanzdaten des Gebisses zu einem ersten Zeitpunkt, wobei ein erstes Bildgebungsvolumen der ersten Magnetresonanzmessung mit einem ersten Volumen des Gebisses abgestimmt ist und eine erste Anzahl von Zähnen umfasst.

In einem weiteren Schritt S2 wird eine Analyse von Abschnitten des Gebisses, welche jeweils eine Teilmenge der Anzahl von Zähnen des Gebisses umfassen, zur Bestimmung einer Auffälligkeit anhand der Magnetresonanzdaten durchgeführt, wobei in zumindest einem Abschnitt eine Auffälligkeit bestimmt wird. Die Magnetresonanzdaten werden hierfür in Abschnitte unterteilt, wobei ein Abschnitt vorzugsweise Magnetresonanzdaten von genau einem Zahn der Anzahl von Zähnen umfasst. Es ist ebenso vorstellbar, dass die Analyse der Abschnitte des Gebisses anhand von Magnetresonanzbildern erfolgt, welche aus den Magnetresonanzdaten rekonstruiert werden. Ein Abschnitt kann dabei z. B. ein Ausschnitt eines Magnetresonanzbilds darstellen, welcher genau einen Zahn der Anzahl von Zähnen umfasst. Die Analyse der Abschnitte erfolgt dabei abschnittsweise, also für jeden einzelnen Abschnitt, bis alle Abschnitte der Magnetresonanzdaten und/oder Magnetresonanzbilder analysiert sind. Die Abschnitte werden vorzugsweise mittels einer geeigneten Bildverarbeitungseinheit analysiert, welche in Abhängigkeit einer Konfiguration eines Prozessors der Bildverarbeitungseinheit jeweils genau einen Abschnitt oder mehrere Abschnitte parallel analysiert. Die Analyse kann beispielsweise eine Korrelation von Kontrasten oder Signalintensitäten von Bildelementen und/oder Volumenelementen der Magnetresonanzbilder mit einem Referenzwert aus einer Datenbank und/oder einem Normalwert der Magnetresonanzbilder umfassen. Der Referenzwert kann beispielsweise ein typischer Kontrast einer Zahnkaries in einem Magnetresonanzbild bei einer gegebenen Bildgebungssequenz sein. Es ist ebenso vorstellbar, dass aus den Kontrasten oder Signalintensitäten mehrerer Bildelemente charakteristische Strukturen abgeleitet werden, welche einer anatomischen Struktur und/oder einer Auffälligkeit zugeordnet werden können. Beispielsweise kann ein Loch in einem Zahn anhand eines unterschiedlichen Kontrasts zu einem Zahnschmelz oder einem Dentin des Zahns mittels der Bildverarbeitungseinheit identifiziert und von einem intakten Teil des betroffenen Zahns unterschieden werden.

In einer Ausführungsform ist der zumindest eine Abschnitt, in welchem eine Auffälligkeit festgestellt wird, ein erster Abschnitt, wobei bei dem Durchführen der Analyse von Abschnitten des Gebisses ein Vorliegen einer Auffälligkeit in einem zweiten Abschnitt ausgeschlossen wird. Dies kann bedeuten, dass bei zumindest einem Zahn des Gebisses eine Auffälligkeit bestimmt wird, während Auffälligkeiten an weiteren Zähnen ausgeschlossen werden. Es kann jedoch ebenso bedeuten, dass bei zumindest einem Zahn des Gebisses eine Auffälligkeit ausgeschlossen wird, während die weiteren Zähne des Gebisses Auffälligkeit aufweisen. Selbstverständlich kann die Anzahl von Zähnen eine Mehrzahl von Abschnitten mit einer Auffälligkeit und eine Mehrzahl von Abschnitten ohne Auffälligkeiten aufweisen, solange zumindest ein Abschnitt mit einer Auffälligkeit bestimmt wird und ein Abschnitt keine Auffälligkeit aufweist.

In einer weiteren Ausführungsform umfasst das Durchführen der Analyse von Abschnitten des Gebisses eine Bestimmung einer Entzündung und/oder einer Zahnkaries. Vorzugsweise wird die Entzündung und/oder die Zahnkaries bei der Analyse der Abschnitte des Gebisses in Abhängigkeit eines Kontrasts oder einer Signalintensität der Magnetresonanzdaten und/oder der Magnetresonanzbilder bestimmt. Die Magnetresonanzmessung kann hierfür mit einer Bildgebungssequenz durchgeführt werden, welche einen hohen Weichgewebekontrast bereitstellt. Ein Beispiel für eine solche Bildgebungssequenz ist eine SE (spin echo) oder eine GRE (gradient echo) Sequenz mit hohen Echozeiten. Insbesondere bei einem Verdacht auf eine Zahnkaries mit Lochbildung kann jedoch auch eine Bildgebungssequenz verwendet werden, welche das Dentin und/oder den Zahnschmelz mit einer hohen Signalintensität abbildet. Mögliche Bildgebungssequenzen können z. B. sehr kurze Echozeiten aufweisen, um einer kurzen T2-Relaxationszeit von Spins des Dentins oder des Zahnschmelzes zu kompensieren. Sehr kurze Echozeiten können z. B. kleiner als 150 µs oder kleiner als 70 µs sein. Mögliche Bildgebungssequenzen stellen z. B. FLASH (fast lowangle shot) oder UTE (ultra-short echo time) Sequenzen dar.

In einem optionalen Schritt S3 wird eine Relativposition zwischen einer Auffälligkeit des zumindest einen Abschnitts und zumindest einem Zahn, welcher den zumindest einen Abschnitt aufweist, bestimmt. Die Bestimmung der Relativposition zwischen der Auffälligkeit des zumindest einen Abschnitts und dem zumindest einen Zahn erfolgt vorzugsweise anhand von Kontrasten oder Signalintensitäten der Magnetresonanzdaten und/oder des Magnetresonanzbilds des zumindest einen Zahns. Beispielsweise können Kontraste von einem oder mehreren Bildelementen eine charakteristische Struktur und/oder eine auffällige Abweichung der Signalintensität gegenüber umliegenden und/oder angrenzenden Bildelementen aufweisen. Insbesondere lässt sich die Kontur des zumindest einen Zahns bei Verwendung einer UTE Sequenz anhand einer charakteristischen Verteilung von signalintensen Bildelementen bestimmen, während sich eine Kontur einer Zahnerkrankung, z. B. aufgrund auffällig geringer Signalintensitäten, von dem zumindest einen Zahn unterscheidet. In anderen Worten kann eine Umfangskontur der Zahnerkrankung anhand von Kontrastunterschieden zu dem zumindest einen Zahn bestimmt werden. Schließlich lassen sich Lageinformationen, wie z. B. die Koordinaten von Punkten an den Umfangskonturen des zumindest einen Zahns und der Zahnerkrankung, miteinander korrelieren, um die Relativposition zwischen dem zumindest einen Zahn und der Zahnerkrankung zu bestimmen.

In einem optionalen Schritt S4 wird eine zweite Magnetresonanzmessung zur Erfassung von zweiten Magnetresonanzdaten des Gebisses des Patienten 15 zu einem zweiten Zeitpunkt durchgeführt, wobei ein zweites Bildgebungsvolumen der zweiten Magnetresonanzmessung mit einem zweiten Volumen des Gebisses abgestimmt ist und zumindest einen Zahn umfasst und wobei zumindest ein Bildgebungsparameter der zweiten Magnetresonanzmessung in Abhängigkeit der Auffälligkeit des ersten Abschnitts bestimmt wird. Wie oben beschrieben, ist der zweite Zeitpunkt dem ersten Zeitpunkt zeitlich nachgelagert. Vorzugsweise wird zumindest ein Bildgebungsparameter der zweiten Magnetresonanzmessung gegenüber der ersten Magnetresonanzmessung verändert, um das zweite Bildgebungsvolumen an das Volumen des Gebisses mit dem ersten Abschnitt anzupassen. Dies kann bedeuten, dass lediglich Magnetresonanzdaten von Abschnitten mit Auffälligkeiten bei der zweiten Magnetresonanzmessung erfasst werden. Wie in Fig. 4 gezeigt, kann das zweite Bildgebungsvolumen der zweiten Magnetresonanzmessung beispielsweise auf sechs Zähne des Gebisses des Patienten 15 beschränkt sein, um die Entwicklung der Zahnkaries und der Entzündung zu quantifizieren, welche bei der Analyse der Abschnitte des Gebisses anhand der ersten Magnetresonanzdaten bestimmt wurden. In Abhängigkeit der Relativposition der betroffenen Zähne kann das zweite Bildgebungsvolumen dabei auf einen einzelnen Zahn, einzelne Gruppen von Zähnen oder alle der sechs Zähne beschränkt sein, um diese mit einer erhöhten Aufnahmequalität zu erfassen. Es ist insbesondere vorstellbar, dass hierfür mehrere Bildgebungssequenzen mit unterschiedlichen zweiten Bildgebungsvolumen durchgeführt werden, welche jeweils an ein Volumen des Gebisses mit dem einzelnen Zahn oder der Gruppe von Zähnen abgestimmt ist. Zumindest ein Bildgebungsparameter der zweiten Magnetresonanzmessung wird dabei in Abhängigkeit der Auffälligkeit des ersten Abschnitts bestimmt. Bezogen auf das Beispiel der Fig. 4 kann dies bedeuten, dass die zweiten Magnetresonanzdaten der Abschnitte mit den Zähnen, welche von einer Entzündung des Zahnfleischs betroffen sind, mit einer zweiten Bildgebungssequenz aufgenommen werden, welche einen hohen Weichgewebekontrast bereitstellt. Bei den Abschnitten mit Zähnen, welche von einer Zahnkaries betroffen sind, wird dagegen eine Echozeit der zweiten Magnetresonanzmessung gegenüber der ersten Magnetresonanzmessung verändert, um den Kontrast des Dentins oder des Zahnschmelzes der betroffenen Zähne zu erhöhen.

In einer Ausführungsform erfolgt das Durchführen der zweiten Magnetresonanzmessung für eine Erfassung von zweiten Magnetresonanzdaten des ersten Abschnitts mit einer zweiten Aufnahmequalität, wobei die zweite Aufnahmequalität höher oder gleich der ersten Aufnahmequalität, welche bei dem Durchführen der ersten Magnetresonanzmessung für eine Erfassung der ersten Magnetresonanzdaten des ersten Abschnitts eingesetzt wird, ist. Vorzugsweise erfolgt die Aufnahme zweiter Magnetresonanzdaten von Zähnen, bei denen eine Zahnerkrankung bei der Analyse der Abschnitte des Gebisses anhand der ersten Magnetresonanzdaten bestimmt wurde, mit einer höheren räumlichen Auflösung. Eine Erhöhung der räumlichen Auflösung der zweiten Magnetresonanzmessung kann beispielsweise durch eine Reduzierung der Schichtdicke vorgenommen werden. Vorzugsweise wird die Erhöhung der räumlichen Auflösung dabei selektiv bei solchen Zähnen vorgenommen, bei denen eine Zahnerkrankung vorliegt.

In einer weiteren Ausführungsform erfolgt das Durchführen der zweiten Magnetresonanzmessung für eine Erfassung von zweiten Magnetresonanzdaten des zweiten Abschnitts mit einer dritten Aufnahmequalität, wobei die dritte Aufnahmequalität niedriger ist als die erste Aufnahmequalität, welche bei dem Durchführen der ersten Magnetresonanzmessung für eine Erfassung der ersten Magnetresonanzdaten des ersten Abschnitts eingesetzt wird. Dies kann bedeuten, dass die Aufnahme zweiter Magnetresonanzdaten von Zähnen ohne Auffälligkeiten mit einer geringeren räumlichen Auflösung erfolgt. Beispielsweise kann die räumliche Auflösung solcher Abschnitte bei der Durchführung der zweiten Magnetresonanzmessung so gering sein, dass eine genaue Quantifizierung des Ausmaßes einer Zahnerkrankung unzweckmäßig ist. Die räumliche Auflösung kann jedoch ausreichend sein, um das Vorliegen einer Zahnerkrankung mittels einer Analyse der Abschnitte der zweiten Magnetresonanzdaten zuverlässig bestimmen zu können.

In einer möglichen Ausführungsform erfolgt das Durchführen der zweiten Magnetresonanzmessung für die Erfassung von zweiten Magnetresonanzdaten des ersten Abschnitts mit einer zweiten Aufnahmequalität und das Durchführen der zweiten Magnetresonanzmessung für die Erfassung von zweiten Magnetresonanzdaten des zweiten Abschnitts mit einer dritten Aufnahmequalität, wobei die zweite Aufnahmequalität höher ist als die dritte Aufnahmequalität. Vorzugsweise weist die zweite Magnetresonanzmessung bei dieser Ausführung eine Mehrzahl von Bildgebungssequenzen auf. Dabei können während einer Bildgebungssequenz der Mehrzahl von Bildgebungssequenzen jeweils zweite Magnetresonanzdaten von genau einem Zahn oder einer Mehrzahl von nebeneinander angrenzenden und/oder übereinander positionierten Zähnen mit einer Auffälligkeit erfasst werden. Die Bildgebungssequenzen können dabei unterschiedliche Bildgebungsvolumen aufweisen, welche an den genau einen Zahn oder die Mehrzahl von nebeneinander und/oder übereinander positionierten Zähnen angepasst sind. Die räumliche Auflösung von Zähnen mit einer Auffälligkeit wird dabei z. B. mittels Anpassung der Schichtdicke gegenüber Zähnen ohne Auffälligkeiten erhöht.

In einer weiteren Ausführungsform werden erste Magnetresonanzbilder anhand der ersten Magnetresonanzdaten erstellt und zweite Magnetresonanzbilder anhand der zweiten Magnetresonanzdaten erstellt, wobei die ersten Magnetresonanzbilder mit den zweiten Magnetresonanzbildern registriert werden. Die Registrierung der ersten Magnetresonanzbilder und der zweiten Magnetresonanzbilder erfolgt **z. B.** mittels rigider oder elastischer Bildregistrierungsverfahren. Vorzugsweise wird die kombinierte Gebissübersichtskarte 40c anhand der registrierten ersten Magnetresonanzbilder und zweiten Magnetresonanzbilder erstellt. Ausschnitte der registrierten Magnetresonanzbilder, wie z. B. Magnetresonanzbilder einzelner Zähne, können dabei zusammen mit der kombinierten Gebissübersichtskarte 40c ausgegeben werden.

In einem weiteren Schritt S5 wird eine Gebissübersichtskarte 40 in Abhängigkeit der Magnetresonanzdaten und der Auffälligkeit des Abschnitts des Gebisses erstellt, wobei die Gebissübersichtskarte 40 eine Repräsentation der Anzahl von Zähnen 41 des Gebisses des Patienten 15 und eine Repräsentation der Auffälligkeit 42 des Abschnitts des Gebisses aufweist. Es ist vorstellbar, dass die Anzahl von Zähnen anhand von Kontrasten oder Signalintensitäten der Magnetresonanzdaten analysiert werden, um einen Typ jedes Zahns der Anzahl von Zähnen zu identifizieren. Für jeden identifizierten Zahn-Typ lässt sich beispielsweise eine entsprechende Repräsentation aus einer Speichereinheit 28 oder einem Cloud-Speicher 30 einlesen und bei der Erstellung der Gebissübersichtskarte 40 verwenden. Es ist aber ebenso vorstellbar, dass die Gebissübersichtskarte 40 anhand von rekonstruierten Magnetresonanzbildern der Magnetresonanzmessung erstellt wird. Anhand der Magnetresonanzbilder lässt sich beispielsweise eine Form und/oder eine Umrisskontur der Zähne ableiten und als Repräsentation verwenden. Die Repräsentationen der Zähne 41 werden bei der Erstellung der Gebissübersichtskarte 40 vorzugsweise geordnet, d. h. in Korrespondenz zu einer anatomisch korrekten Anordnung der Zähne, dargestellt. Die Zähne können weiterhin mit einer Nummerierung 52 und/oder einer Kennzeichnung 53 versehen sein, welche beispielsweise Informationen über einen Typ und/oder eine Position eines Zahns in dem Gebiss des Patienten 15 umfassen. Gleichermaßen kann die Repräsentation der Auffälligkeit 42 anhand einer identifizierten Form und/oder einer identifizierten Umrisskontur erstellt werden oder aus einem Datenspeicher eingelesen werden. Es ist vorstellbar, dass die Repräsentation der Auffälligkeit 42, wie in den Figuren 2 bis 5 gezeigt, Markierungen 54 und/oder Hinweise 51 aufweisen, welche eine Information über einen Typ, eine Position und/oder ein Ausmaß einer Zahnerkrankung angeben.

In einer möglichen Ausführungsform erfolgt die Erstellung der Gebissübersichtskarte 40 in Abhängigkeit der Relativposition zwischen der Auffälligkeit eines Abschnitts und dem zumindest einen Zahn des Abschnitts. Wie in Fig. 5 gezeigt, wird die Repräsentation einer Zahnerkrankung 42 bei dem Erstellen der kombinierten Gebissübersichtskarte 40c relativ zu der Repräsentation des Zahns 41 positioniert, sodass eine Position der Repräsentation der Zahnerkrankung 42d zumindest näherungsweise mit einer anatomisch korrekten Position der Zahnerkrankung an dem Zahn übereinstimmt.

In einem optionalen Schritt S6 wird eine Abweichung zwischen den ersten Magnetresonanzdaten und den zweiten Magnetresonanzdaten bestimmt, wobei eine Information über die Abweichung zusammen mit der Gebissübersichtskarte 40 ausgegeben wird. Die Bestimmung der Abweichung erfolgt insbesondere anhand einer Differenz von Kontrastwerten oder Signalintensitätswerten der ersten Magnetresonanzdaten und der zweiten Magnetresonanzdaten. Es ist jedoch ebenso vorstellbar, dass die Bestimmung der Abweichung anhand von Kontrasten von Bildelementen von ersten Magnetresonanzbildern und zweiten Magnetresonanzbildern, welche aus den ersten Magnetresonanzdaten und den zweiten Magnetresonanzdaten rekonstruiert werden, erfolgt. Die Bestimmung der Abweichung kann beispielsweis eine Korrelation von Lageinformationen eines Zahns oder einer Auffälligkeit umfassen. Anhand der Abweichung der Lageinformation zwischen dem ersten Zeitpunkt und dem zweiten Zeitpunkt kann eine Entwicklung einer Zahnerkrankung oder einer Zahnstellung abgeleitet werden, welche beispielsweise in Form eines Hinweises 51, einer Markierung 54 und/oder einer Repräsentation der Auffälligkeit 42 auf der kombinierten Gebissübersichtskarte 40c dargestellt wird. Wie in Fig. 5 gezeigt, kann eine Repräsentation der Zahnkaries 42a zu dem ersten Zeitpunkt mit einer Repräsentation der Zahnkaries 42b zu dem zweiten Zeitpunkt überlagert werden, um die Entwicklung der Zahnerkrankung darzustellen.

In einem weiteren Schritt S7 des Verfahrens wird die Gebissübersichtskarte 40 bereitgestellt. Es ist vorstellbar, dass die Gebissübersichtskarte 40 bei dem Bereitstellen an eine Speichereinheit 29 und/oder einen Cloud-Speicher 30 übertragen wird. Es ist ebenso vorstellbar, dass die Gebissübersichtskarte 40 im Rahmen einer Beurteilung des Zustands des Gebisses des Patienten 15 an eine Anzeigeeinheit 24 der Magnetresonanzvorrichtung 10 übertragen wird. Die Gebissübersichtskarte 40 kann ferner an eine Anzeigeeinheit oder eine Recheneinheit eines Mobilgeräts des Patienten 15 ausgegeben werden. Die Recheneinheit des Mobilgeräts kann dazu ausgelegt sein, die Gebissübersichtskarte 40 mittels einer dedizierten Software-Anwendung zu verarbeiten, z. B. um eine Zahnreinigung des Gebisses zu verbessern. Das Bereitstellen der Gebissübersichtskarte 40 an die Anzeigeeinheit 24, die Speichereinheit 29, den Cloud-Speicher 30 und/oder das Mobilgerät des Patienten 15 kann dabei kabelgebunden oder kabellos mittels geeigneter Schnittstellen erfolgen.

Natürlich sind die hier beschriebenen Ausführungsformen des erfindungsgemäßen Verfahrens und der erfindungsgemäßen Ultraschallaufnahmevorrichtung als beispielhaft zu verstehen. Einzelne Ausführungsformen sind daher um Merkmale anderer Ausführungsformen erweiterbar. Insbesondere ist die Reihenfolge der Verfahrensschritte des erfindungsgemäßen Verfahrens als beispielhaft zu verstehen. Die einzelnen Schritte können auch in einer anderen Reihenfolge durchgeführt werden oder sich zeitlich teilweise oder vollständig überschneiden.

## Patentansprüche

1. Magnetresonanzvorrichtung (10) mit einer Recheneinheit (28), wobei die Recheneinheit (28) dazu ausgelegt ist, eine Gebissübersichtskarte (40) von einem Gebiss eines Untersuchungsobjekts anhand von Magnetresonanzdaten einer Magnetresonanzmessung des Gebisses gemäß den folgenden Schritten zu erstellen:
• Durchführen (S1) einer Magnetresonanzmessung zur Erfassung von Magnetresonanzdaten des Gebisses, wobei ein Bildgebungsvolumen der Magnetresonanzmessung mit einem Volumen des Gebisses abgestimmt ist und wobei das Bildgebungsvolumen eine Anzahl von Zähnen des Gebisses umfasst,
• Durchführen (S2) einer Analyse von Abschnitten des Gebisses, welche jeweils eine Teilmenge der Anzahl von Zähnen des Gebisses umfassen, zur Bestimmung einer Auffälligkeit anhand der Magnetresonanzdaten, wobei in zumindest einem Abschnitt eine Auffälligkeit bestimmt wird, wobei die Magnetresonanzmessung (S1) eine erste Magnetresonanzmessung ist, wobei die erste Magnetresonanzmessung zur Erfassung von ersten Magnetresonanzdaten des Gebisses zu einem ersten Zeitpunkt durchgeführt wird, wobei ein erstes Bildgebungsvolumen der ersten Magnetresonanzmessung mit einem ersten Volumen des Gebisses abgestimmt ist und eine erste Anzahl von Zähnen umfasst und wobei der zumindest eine Abschnitt, in welchem die Auffälligkeit bestimmt wird, ein erster Abschnitt ist, wobei in einem weiteren Schritt (S4) eine zweite Magnetresonanzmessung zur Erfassung von zweiten Magnetresonanzdaten des Gebisses zu einem zweiten Zeitpunkt durchgeführt wird, wobei ein zweites Bildgebungsvolumen der zweiten Magnetresonanzmessung mit einem zweiten Volumen des Gebisses abgestimmt ist und zumindest einen Zahn umfasst und wobei zumindest ein Bildgebungsparameter der zweiten Magnetresonanzmessung in Abhängigkeit der Auffälligkeit des ersten Abschnitts bestimmt wird,
• Erstellen (S5) einer zweiten Gebissübersichtskarte (40) in Abhängigkeit der zweiten Magnetresonanzdaten und der Auffälligkeit des zumindest einen Abschnitts des Gebisses, wobei die Gebissübersichtskarte (40) eine Repräsentation eines Zahns (41) des Gebisses des Untersuchungsobjekts sowie eine Repräsentation der Auffälligkeit (42) des zumindest einen Abschnitts des Gebisses aufweist,
• Bereitstellen (S7) der Gebissübersichtskarte (40).

2. Magnetresonanzvorrichtung (10) nach Anspruch 1, wobei der zumindest eine Abschnitt, in welchem die Auffälligkeit bestimmt wird, ein erster Abschnitt ist und wobei bei dem Durchführen (S2) der Analyse von Abschnitten des Gebisses ein Vorliegen einer Auffälligkeit in einem zweiten Abschnitt ausgeschlossen wird.

3. Magnetresonanzvorrichtung (10)nach einem der Ansprüche 1 oder 2, wobei das Durchführen (S2) der Analyse von Abschnitten des Gebisses eine Bestimmung einer Entzündung und/oder einer Zahnkaries umfasst.

4. Magnetresonanzvorrichtung (10)nach einem der vorhergehenden Ansprüche, ferner aufweisend den Schritt:
• Bestimmen (S3) einer Relativposition zwischen einer Auffälligkeit des zumindest einen Abschnitts und zumindest einem Zahn, welcher den zumindest einen Abschnitt aufweist,
wobei das Erstellen (S5) der Gebissübersichtskarte (40) in Abhängigkeit der Relativposition zwischen der Auffälligkeit des zumindest einen Abschnitts und dem zumindest einen Zahn erfolgt.

5. Magnetresonanzvorrichtung (10)nach einem der vorhergehenden Ansprüche, wobei das Durchführen (S4) der zweiten Magnetresonanzmessung für eine Erfassung von zweiten Magnetresonanzdaten des ersten Abschnitts mit einer zweiten Aufnahmequalität erfolgt, wobei die zweite Aufnahmequalität höher oder gleich einer ersten Aufnahmequalität, welche bei dem Durchführen der ersten Magnetresonanzmessung für eine Erfassung der ersten Magnetresonanzdaten des ersten Abschnitts eingesetzt wird, ist.

6. Magnetresonanzvorrichtung (10)nach einem der Ansprüche 1 bis 4, wobei das Durchführen (S4) der zweiten Magnetresonanzmessung für eine Erfassung von zweiten Magnetresonanzdaten eines zweiten Abschnitts mit einer dritten Aufnahmequalität erfolgt, wobei die dritte Aufnahmequalität niedriger ist als eine erste Aufnahmequalität, welche bei dem Durchführen der ersten Magnetresonanzmessung für eine Erfassung der ersten Magnetresonanzdaten des ersten Abschnitts eingesetzt wird.

7. Magnetresonanzvorrichtung (10)nach einem der Ansprüche 1 bis 4, wobei das Durchführen (S4) der zweiten Magnetresonanzmessung für die Erfassung von zweiten Magnetresonanzdaten des ersten Abschnitts mit einer zweiten Aufnahmequalität erfolgt und das Durchführen (S4) der zweiten Magnetresonanzmessung für die Erfassung von zweiten Magnetresonanzdaten eines zweiten Abschnitts mit einer dritten Aufnahmequalität erfolgt, wobei die zweite Aufnahmequalität höher ist als die dritte Aufnahmequalität.

8. Magnetresonanzvorrichtung (10)nach einem der Ansprüche 1 bis 5, wobei das zweite Bildgebungsvolumen der zweiten Magnetresonanzmessung auf den ersten Abschnitt des Gebisses begrenzt ist.

9. Magnetresonanzvorrichtung (10)nach einem der vorhergehenden Ansprüche, wobei das Durchführen (S4) der zweiten Magnetresonanzmessung zur Aufnahme von zweiten Magnetresonanzdaten mit einer zweiten Bildgebungssequenz erfolgt, wobei die zweite Bildgebungssequenz in Abhängigkeit der Auffälligkeit des ersten Abschnitts bestimmt wird.

10. Magnetresonanzvorrichtung (10)nach einem der vorhergehenden Ansprüche, wobei erste Magnetresonanzbilder anhand der ersten Magnetresonanzdaten erstellt werden und wobei zweite Magnetresonanzbilder anhand der zweiten Magnetresonanzdaten erstellt werden und wobei die ersten Magnetresonanzbilder mit den zweiten Magnetresonanzbildern registriert werden.

11. Magnetresonanzvorrichtung (10)nach einem der vorhergehenden Ansprüche, ferner aufweisend den Schritt:
• Bestimmen (S6) einer Abweichung zwischen den ersten Magnetresonanzdaten und den zweiten Magnetresonanzdaten, wobei eine Information über die Abweichung zusammen mit der Gebissübersichtskarte (40) ausgegeben wird.

12. Computerprogrammprodukt, welches direkt in einen Datenspeicher einer Recheneinheit (28) einer Magnetresonanzvorrichtung (10) nach einem der vorhergehenden Ansprüche ladbar ist, mit Programmcode-Mitteln, um eine Gebissübersichtskarte (40) von einem Gebiss eines Untersuchungsobjekts anhand von Magnetresonanzdaten einer Magnetresonanzmessung des Gebisses zu erstellen, wenn das Computerprogrammprodukt in der Recheneinheit (28) der Magnetresonanzvorrichtung (10) die Schritte der Recheneinheit (28) gemäß einem der vorherigen Ansprüche ausführt.

## Claims

1. Magnetic resonance apparatus (10) with a computing unit (28), wherein the computing unit (28) is configured to compile a dental overview map (40) of a dentition of an examination object on the basis of magnetic resonance data from a magnetic resonance measurement of the dentition, according to the following steps:
• performing (S1) a magnetic resonance measurement for acquiring magnetic resonance data from the dentition, wherein an imaging volume of the magnetic resonance measurement is matched with a volume of the dentition and wherein the imaging volume includes a number of teeth in the dentition,
• performing (S2) an analysis of sections of the dentition which each include a subset of the number of teeth in the dentition in order to determine an abnormality on the basis of the magnetic resonance data, wherein an abnormality is determined in at least one section, wherein the magnetic resonance measurement (S1) is a first magnetic resonance measurement, wherein the first magnetic resonance measurement for acquiring first magnetic resonance data from the dentition is performed at a first time point, wherein a first imaging volume of the first magnetic resonance measurement is matched with a first volume of the dentition and includes a first number of teeth and wherein the at least one section in which the abnormality is determined is a first section, wherein in a further step (S4), a second magnetic resonance measurement for acquiring second magnetic resonance data from the dentition is performed at a second time point, wherein a second imaging volume of the second magnetic resonance measurement with a second volume of the dentition and includes at least one tooth and wherein at least one imaging parameter of the second magnetic resonance measurement is determined as a function of the abnormality of the first section,
• compiling (S5) a second dental overview map (40) as a function of the second magnetic resonance data and the abnormality of the at least one section of the dentition, wherein the dental overview map (40) comprises a representation of a tooth (41) of the dentition of the examination object and a representation of the abnormality (42) of the at least one section of the dentition,
• providing (S7) the dental overview map (40).

2. Magnetic resonance apparatus (10) according to claim 1, wherein the at least one section in which the abnormality is determined is a first section and wherein, during the performance (S2) of the analysis of sections of the dentition, the presence of an abnormality in a second section is excluded.

3. Magnetic resonance apparatus (10) according to one of claims 1 or 2, wherein the performance (S2) of the analysis of sections of the dentition includes the determination of inflammation and/or dental caries.

4. Magnetic resonance apparatus (10) according to one of the preceding claims further having the step:
• determining (S3) a relative position between an abnormality of the at least one section and at least one tooth with the at least one section,
wherein the compilation (S5) of the dental overview map (40) takes place as a function of the relative position between the abnormality of the at least one section and the at least one tooth.

5. Magnetic resonance apparatus (10) according to one of the preceding claims, wherein the performance (S4) of the second magnetic resonance measurement for acquiring second magnetic resonance data from the first section takes place with a second recording quality, wherein the second recording quality is higher than or equal to the one first recording quality used during the performance of the first magnetic resonance measurement for the acquisition of the first magnetic resonance data from the first section.

6. Magnetic resonance apparatus (10) according to one of claims 1 to 4, wherein the performance (S4) of the second magnetic resonance measurement for acquiring second magnetic resonance data from a second section takes place with a third recording quality, wherein the third recording quality is lower than a first recording quality used during the performance of the first magnetic resonance measurement for the acquisition of the first magnetic resonance data from the first section.

7. Magnetic resonance apparatus (10) according to one of claims 1 to 4, wherein the performance (S4) of the second magnetic resonance measurement for the acquisition of second magnetic resonance data from the first section takes place with a second recording quality and the performance (S4) of the second magnetic resonance measurement for the acquisition of second magnetic resonance data from a second section takes place with a third recording quality, wherein the second recording quality is higher than the third recording quality.

8. Magnetic resonance apparatus (10) according to one of claims 1 to 5, wherein the second imaging volume of the second magnetic resonance measurement is restricted to the first section of the dentition.

9. Magnetic resonance apparatus (10) according to one of the preceding claims, wherein the performance (S4) of the second magnetic resonance measurement for recording second magnetic resonance data takes place with a second imaging sequence, wherein the second imaging sequence is determined as a function of the abnormality of the first section.

10. Magnetic resonance apparatus (10) according to one of the preceding claims, wherein first magnetic resonance images are compiled on the basis of the first magnetic resonance data and wherein second magnetic resonance images are compiled on the basis of the second magnetic resonance data and wherein the first magnetic resonance images are registered with the second magnetic resonance images.

11. Magnetic resonance apparatus (10) according to one of the preceding claims, further comprising the step:
• determining (S6) a deviation between the first magnetic resonance data and the second magnetic resonance data, wherein information on the deviation is output together with the dental overview map (40).

12. Computer program product which can be loaded directly into a data storage device of a computing unit (28) of a magnetic resonance apparatus (10) according to one of the preceding claims, with program code means for compiling a dental overview map of the dentition of an examination object on the basis of magnetic resonance data from a magnetic resonance measurement of the dentition, when the computer program product carries out the steps of the computing unit (28) according to one of the preceding claims in the computing unit (28) of the magnetic resonance apparatus (10).

## Revendications

1. Dispositif (10) de résonance magnétique comprenant une unité (28) informatique, dans lequel l'unité (28) informatique est conçue pour établir une carte (40) synoptique d'une dentition d'un objet à étudier, à l'aide de données de résonance magnétique d'une mesure par résonance magnétique de la dentition selon les stades suivants :
• effectuer (S1) une mesure par résonance magnétique pour la détection de données de résonance magnétique de la dentition, dans lequel un volume d'imagerie de la mesure par résonance magnétique est déterminé par un volume de la dentition et dans lequel le volume d'imagerie comprend un nombre de dents de la dentition,
• effectuer (S2) une analyse de segments de la dentition, qui comprennent chacun une quantité partielle du nombre de dents de la dentition, pour la détermination d'une singularité, à l'aide des données de résonance magnétique, dans lequel on détermine une singularité dans au moins un segment, dans lequel la mesure (S1) par résonance magnétique est une première mesure par résonance magnétique, dans lequel la première mesure par résonance magnétique est effectuée pour la détermination de premières données de résonance magnétique de la dentition à un premier instant, dans lequel un premier volume d'imagerie de la première mesure par résonance magnétique est déterminé par un premier volume de la dentition et comprend un premier nombre de dents et dans lequel le au moins un segment, dans lequel la singularité est déterminée, est un premier segment, dans lequel, dans un autre stade (S4), on effectue une deuxième mesure par résonance magnétique pour la détection de deuxièmes données de résonance magnétique de la dentition à un deuxième instant, dans lequel un deuxième volume d'imagerie de la deuxième mesure par résonance magnétique est déterminé par un deuxième volume de la dentition et comprend au moins une dent, et dans lequel au moins un paramètre d'imagerie est adapté à la deuxième mesure par résonance magnétique en fonction de la singularité du premier segment,
• établir (S5) une deuxième carte (40) synoptique de la dentition en fonction des deuxièmes données de résonance magnétique et de la singularité du au moins un segment de la dentition, dans lequel la carte (40) synoptique de la dentition a une représentation d'une dent (41) de la dentition de l'objet à étudier, ainsi qu'une représentation de la singularité (42) du au moins un segment de la dentition,
• mettre (S7) à disposition la carte (40) synoptique de la dentition.

2. Dispositif (10) de résonance magnétique suivant la revendication 1, dans lequel le au moins un segment, dans lequel on détermine la singularité, est un premier segment et dans lequel, lors de l'exécution (S2) de l'analyse de segments de la dentition, on exclut la présence d'une singularité dans un deuxième segment.

3. Dispositif (10) de résonance magnétique suivant l'une des revendications 1 ou 2, dans lequel effectuer (S2) l'analyse de segments de la dentition comprend une détermination d'une inflammation ou d'une carie de dent.

4. Dispositif (10) de résonance magnétique suivant l'une des revendications précédentes, comprenant en outre le stade :
• détermination (S3) d'une position relative entre une singularité du au moins un segment et au moins une dent, qui a le au moins un segment,
dans lequel l'établissement (S5) de la carte (40) synoptique de la dentition s'effectue en fonction de la position relative entre la singularité du au moins un segment et la au moins une dent.

5. Dispositif (10) de résonance magnétique suivant l'une des revendications précédentes, dans lequel effectuer (S4) la deuxième mesure par résonance magnétique pour une détection de deuxièmes données de résonance magnétique du premier segment s'effectue avec une deuxième qualité d'enregistrement, dans lequel la deuxième qualité d'enregistrement est supérieure ou égale à une première qualité d'enregistrement, qui est utilisée lorsque l'on effectue la première mesure par résonance magnétique pour une détection des premières données de résonance magnétique du premier segment.

6. Dispositif (10) de résonance magnétique suivant l'une des revendications 1 à 4, dans lequel effectuer (S4) la deuxième mesure par résonance magnétique pour une détection de deuxièmes données de résonance magnétique d'un deuxième segment s'effectue avec une troisième qualité d'enregistrement, dans lequel la troisième qualité d'enregistrement est inférieure à une première qualité d'enregistrement, qui est utilisée lors de l'exécution de la première mesure par résonance magnétique pour une détection des premières données de résonance magnétique du premier segment.

7. Dispositif (10) de résonance magnétique suivant l'une des revendications 1 à 4, dans lequel effectuer (S4) la deuxième mesure par résonance magnétique pour la détection de deuxièmes données de résonance magnétique du premier segment s'effectue à une deuxième qualité d'enregistrement et effectuer (S4) la deuxième mesure de résonance magnétique pour la détection des deuxièmes données de résonance magnétique d'un deuxième segment s'effectue à une troisième qualité d'enregistrement, dans lequel la deuxième qualité d'enregistrement est supérieure à la première qualité d'enregistrement.

8. Dispositif (10) de résonance magnétique suivant l'une des revendications 1 à 5, dans lequel le deuxième volume d'imagerie de la deuxième mesure par résonance magnétique est limité au premier segment de la dentition.

9. Dispositif (10) de résonance magnétique suivant l'une des revendications précédentes, dans lequel effectuer (S4) la deuxième mesure par résonance magnétique pour l'enregistrement de deuxièmes données de résonance magnétique s'effectue par une deuxième séquence d'imagerie, dans lequel la deuxième séquence d'imagerie est déterminée en fonction de la singularité du premier segment.

10. Dispositif (10) de résonance magnétique suivant l'une des revendications précédentes, dans lequel on établit de premières images de résonance magnétique à l'aide des premières données de résonance magnétique et dans lequel on établit des deuxièmes images de résonance magnétique à l'aide des deuxièmes données de résonance magnétique et dans lequel on met les premières images de résonance magnétique en correspondance avec les deuxièmes images de résonance magnétique.

11. Dispositif (10) de résonance magnétique suivant l'une des revendications précédentes, comprenant en outre le stade :
• détermination (S6) d'un écart entre les premières données de résonance magnétique et les deuxièmes données de résonance magnétique, dans lequel on donne une information sur l'écart ensemble avec la carte (40) synoptique de la dentition.

12. Produit de programme d'ordinateur, qui peut être chargé directement dans une mémoire de données d'une unité (28) informatique d'un dispositif (10) de résonance magnétique suivant l'une des revendications précédentes, comprenant des moyens de code de programme pour établir une carte (40) synoptique d'une dentition d'un objet à examiner, à l'aide de données de résonance magnétique d'une mesure par résonance magnétique de la dentition, lorsque le produit de programme d'ordinateur exécute dans l'unité (28) informatique du dispositif (10) de résonance magnétique les stades de l'unité (28) informatique suivant l'une des revendications précédentes.
